# EUROPEAN PATENT APPLICATION

(11) **EP 4 623 916 A1**
(43) Date of publication of application: **01.10.2025**
(21) Application number: 23894559.6
(22) Date of filing: 20.11.2023
(51) Int. Cl.: A61K 31/506, A61K 9/32, A61K 47/02, A61K 47/04, A61K 47/10, A61K 47/12, A61K 47/14, A61K 47/20, A61K 47/26, A61K 47/32, A61K 47/38, A61K 47/44, A61P 17/00, A61P 17/04, A61P 25/00

(54) **ORAL SOLID FORMULATION**

(30) Priority: 21.11.2022 JP 2022185858
(71) Applicant: MOCHIDA PHARMACEUTICAL CO., LTD., Tokyo 160-8515 (JP)
(72) Inventor: SHIMADA Shunsuke, Tokyo 160-8515 (JP); KAWAMOTO Masaki, Tokyo 160-8515 (JP); MAEDA Yasuhiro, Tokyo 160-8515 (JP); NISHIMURA Masahiro, Tokyo 160-8515 (JP); YURA Takayuki, Tokyo 160-8515 (JP); KOSHIMO Takeshi, Tokyo 160-8515 (JP)
(74) Representative: Hoffmann Eitle
(86) International application number: PCT/JP2023/041643
(87) International publication number: WO 2024/111552

(57) **Abstract**

The present invention provides an oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient. The present invention relates to an oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof.

## Description

### Technical Field

The present invention relates to an oral solid formulation containing 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof.

### Background Art

1-((1R,2R)-2-hydroxy-4,4-dimethyl-1,2,3,4-tetrahydronaphthalen-1-yl)-3-(2-phenylpyridin-3-yl)urea is a compound represented by the chemical structural formula (I) (hereinafter sometimes referred to as compound (I)).

1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, is a compound having excellent TrkA (Tropomyosin receptor kinase A) inhibitory activity; it is known to be useful for the prevention or/and treatment of diseases in which TrkA is involved (e.g., pain, pruritus, etc.) (Patent Literature 1: WO 2018/199166 pamphlet).

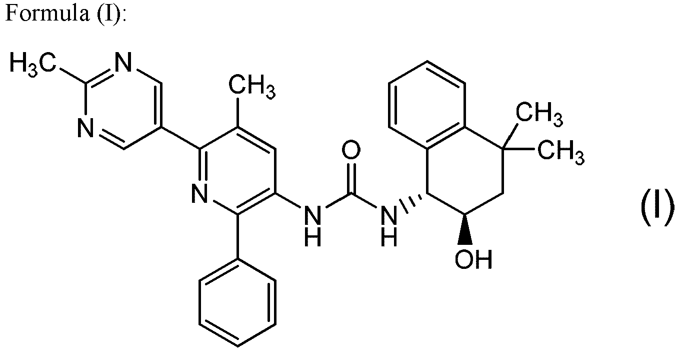

Patent Literature 1 discloses that a pharmaceutical composition of compound (I) can be optionally combined with pharmaceutically acceptable additives (e.g., excipients, binders, glidants, disintegrants, surfactants, flow agents, coating agents, plasticizers, masking agents, coloring agents, flavoring agents, antiseptics, isotonic agents, pH adjusters, stabilizers, dispersants, antioxidants, buffers, preservatives, fragrances, solubilizers, absorption enhancers, gelling agents, suspending agents, emulsifying agents, generally used suitable additives, or solvents) to form various dosage forms (e.g., tablets, capsules, granules, powders, pills, aerosols, inhalants, ointments, patches, suppositories, injections, troches, solutions, spirits, suspensions, extracts, elixirs, etc.). However, specific formulation prescriptions and formulations prepared using specific additives, solvents, etc., are not disclosed.

For the purpose of improving patient medication compliance and providing economical medical care, for example, orally administered formulations such as tablets and capsules that are easy to take, formulations that can achieve efficacy with a reduced number of doses per day, and the like are required in medical settings.

### Citation List

### Patent Literatures

Patent Literature 1: WO 2018/199166 pamphlet

### Summary of Invention

An object of the present invention is to provide an oral solid formulation containing 1-((1R,2R)-2-hydroxy-4,4-dimethyl-1,2,3,4-tetrahydronaphthalen-1-yl)-3-(2-phenylpyridin-3-yl)urea (compound (I)), or a pharmaceutically acceptable salt thereof, or a solvate thereof, as an active ingredient.

Another object of the present invention is to provide an oral solid formulation capable of suppressing a rapid increase in the blood concentration of the active ingredient compound at an early stage after administration of the formulation to a subject.

It has been found that compound (I), when orally administered to a subject, dissolves rapidly in the stomach (under acidic conditions of pH=about 1 to about 2), is absorbed, shows a rapid rise in blood concentration at an early stage after administration, and has the property of being rapidly eliminated.

In the case of compound (I), which shows a rapid increase in blood concentration at an early stage after administration and is rapidly eliminated before the next administration, resulting in large fluctuations in blood concentration, there is a concern about the possibility of adverse effects such as the manifestation of toxicity associated with the temporarily high blood concentration of compound (I).

Under such circumstances, it has become urgent to provide a formulation containing compound (I), or a pharmaceutically acceptable salt thereof, or a solvate thereof, as an active ingredient, which can suppress the manifestation of toxicity.

As a result of intensive studies to achieve the above object, the present inventors have designed a formulation in which a core containing 1-((1R,2R)-2-hydroxy-4,4-dimethyl-1,2,3,4-tetrahydronaphthalen-1-yl)-3-(2-phenylpyridin-3-yl)urea (compound (I)), or a pharmaceutically acceptable salt thereof, or a solvate thereof, as an active ingredient is coated with a specific coating agent; when the formulation prepared according to this design was administered, it became possible to suppress the rapid increase in the blood concentration of compound (I) at an early stage after administration to a subject, leading the inventors to discover an oral solid formulation.

The inventors have further found that by using a coating agent in which the formulation begins to dissolve at a specific pH, it is possible to obtain an oral solid formulation with excellent sustainability of the blood concentration of compound (I). Specific examples of the oral solid formulation include a formulation in which a core containing 50 mg or 150 mg of compound (I) is coated with a coating agent in which the formulation begins to dissolve at pH=about 5.0 to about 6.5 or pH=about 5.0 to about 7.0; 12 hours or 24 hours after administration of the formulation to a subject, the blood concentration of compound (I) is about 100 nM (preferably 90-110 nM) or more.

Thus, the present inventors have found (i) an oral solid formulation containing compound (I) which is considered to be highly safe, and (ii) an oral solid formulation in which the blood concentration of compound (I) is sustained for a long time when the formulation containing compound (I) is administered to a subject, particularly an oral solid formulation which can be expected to have a long-lasting efficacy by administration once or twice a day, thereby completing the present invention.

Since compound (I) contained in the oral solid formulation of the present invention has TrkA inhibitory activity, administration of this formulation can have an ameliorating effect on diseases in which TrkA is involved (e.g., pain, pruritus, etc.).

The present invention is an oral solid formulation containing 1-((1R,2R)-2-hydroxy-4,4-dimethyl-1,2,3,4-tetrahydronaphthalen-1-yl)-3-(2-phenylpyridin-3-yl)urea or a pharmaceutically acceptable salt thereof, or a solvate thereof as shown in the following aspects, more specifically, as follows [1] to [21-3].

[1] An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
   (1) a core containing the active ingredient, and
   (2) a coating layer covering the core, the coating layer containing at least one coating agent selected from, for example, hypromellose phthalate, methacrylic acid copolymer, hypromellose acetate succinate, cellulose acetate phthalate, and carboxymethyl ethyl cellulose.
[1-1] In aspect [1], preferably, the coating agent for coating the core is at least one selected from the group consisting of hypromellose phthalate, methacrylic acid copolymer, and hypromellose acetate succinate; more preferably, one selected from the group consisting of hypromellose phthalate, methacrylic acid copolymer, and hypromellose acetate succinate.
[1-2] In aspect [1], preferably, the coating agent for coating the core is at least one selected from the group consisting of hypromellose phthalate, methacrylic acid copolymer, hypromellose acetate succinate, cellulose acetate phthalate, and carboxymethyl ethyl cellulose; more preferably one selected from the group consisting of hypromellose phthalate, methacrylic acid copolymer, hypromellose acetate succinate, cellulose acetate phthalate, and carboxymethyl ethyl cellulose.
[1-3] In aspects [1] to [1-2], the methacrylic acid copolymer is, for example, methacrylic acid copolymer LD, methacrylic acid copolymer L, methacrylic acid copolymer S, dry methacrylic acid copolymer LD, etc.; preferably, the methacrylic acid copolymer is at least one selected from the group consisting of methacrylic acid copolymer LD, methacrylic acid copolymer L, methacrylic acid copolymer S, and dry methacrylic acid copolymer LD; more preferably, one selected from the group consisting of methacrylic acid copolymer LD, methacrylic acid copolymer L, methacrylic acid copolymer S, and dry methacrylic acid copolymer LD.
[2] The oral solid formulation according to any one of aspects [1] to [1-3], wherein the coating layer further contains at least one additive selected from, for example, a plasticizer, an excipient, a binder, a disintegrant, a lubricant, a surfactant, a pH adjuster, a solubilizer, a stabilizer, an antioxidant, an adsorbent, a corrigent, a sweetener, a coloring agent, a flavoring agent, a flow agent, a glidant, a coating agent other than the coating agent described in aspect [1] (including a second coating agent) and the like.
[2-1] In aspect [2], the plasticizer is at least one selected from, for example, triethyl citrate, tributyl citrate, Macrogol (registered trademark) (polyethylene glycol), propylene glycol, sodium lauryl sulfate in ethanol, triacetin, polysorbate 80, etc.; preferably, at least one selected from the group consisting of triethyl citrate, Macrogol (registered trademark) (polyethylene glycol), propylene glycol, triacetin, and polysorbate 80; more preferably triethyl citrate.
[2-2] In aspect [2], the second coating agent is at least one selected from, for example, titanium dioxide, talc, crystalline cellulose, calcium carbonate, shellac, etc.; preferably, at least one selected from the group consisting of titanium dioxide, talc, crystalline cellulose, calcium carbonate, and shellac; more preferably, titanium dioxide and/or talc.
[3] The oral solid formulation according to any one of aspects [1] to [2-2], wherein a component that can be contained in the core is at least one selected from additives such as excipients, flow agents, disintegrants, and glidants.
[3-1] The oral solid formulation according to any one of aspects [1] to [2-2], wherein a component that can be contained in the core is at least one additive selected from the group consisting of excipients, flow agents, disintegrants, and glidants.
[3-2] The oral solid formulation according to any one of aspects [1] to [2-2], wherein the core contains an excipient, a flow agent, a disintegrant, and a glidant.
[4] The oral solid formulation according to any one of aspects [3] to [3-2], wherein the excipient is at least one selected from, for example, glucose, lactose (monohydrate, spray-dried monohydrate, anhydrous, etc.), granulated lactose, sucrose, white sugar, mannitol (D-mannitol), mannite, xylitol, sorbitol (D-sorbitol), crystalline cellulose, microcrystalline cellulose, silicic acid, starch, maize starch, potato starch, dicalcium phosphate dihydrate, isomalt, anhydrous calcium phosphate, anhydrous calcium hydrogen phosphate, corn starch, pregelatinized starch, partially pregelatinized starch, light anhydrous silicic acid, titanium dioxide, etc.
[4-1] In aspect [4], preferably, the excipient is at least one selected from the group consisting of lactose (monohydrate, spray-dried monohydrate, anhydrous, etc.), granulated lactose, white sugar, mannitol (D-mannitol), crystalline cellulose, microcrystalline cellulose, silicic acid, starch, maize starch, potato starch, anhydrous calcium hydrogen phosphate, corn starch, pregelatinized starch and partially pregelatinized starch; more preferably at least one selected from the group consisting of crystalline cellulose, mannitol (D-mannitol) and granulated lactose; further preferably one selected from the group consisting of mannitol, crystalline cellulose, a mixture of mannitol and crystalline cellulose, and a mixture of crystalline cellulose and granulated lactose.
[5] The oral solid formulation according to any one of aspects [3] to [3-2], wherein the flow agent is at least one selected from, for example, silicon dioxide (light anhydrous silicic acid, silica gel), talc, hydrous silicon dioxide, magnesium aluminometasilicate, and calcium hydrogen phosphate granulate.
[5-1] In aspect [5], preferably, the flow agent is at least one selected from the group consisting of light anhydrous silicic acid, talc, and magnesium aluminometasilicate; more preferably one selected from the group consisting of light anhydrous silicic acid, a mixture of light anhydrous silicic acid and talc, talc, and magnesium aluminometasilicate.
[6] The oral solid formulation according to any one of aspects [3] to [3-2], wherein the disintegrant is at least one selected from, for example, starches (maize starch, potato starch, starch, pregelatinized starch, partially pregelatinized starch), sodium carboxymethyl starch, carmellose (carboxymethyl cellulose, CMC), carmellose calcium, croscarmellose sodium, crospovidone, sodium starch glycolate, sodium carboxymethyl cellulose, carboxymethyl cellulose calcium, polyvinylpyrrolidone (PVPP), methylcellulose, microcrystalline cellulose, crystalline cellulose, low-alkyl-substituted hydroxypropyl cellulose (L-HPC), sodium alginate, corn starch, and sodium carboxymethyl starch.
[6-1] In aspect [6], preferably, the disintegrant is at least one selected from the group consisting of sodium carboxymethyl starch, carmellose (carboxymethyl cellulose, CMC), croscarmellose sodium, crospovidone, sodium starch glycolate, carboxymethyl cellulose calcium, polyvinylpyrrolidone (PVPP), microcrystalline cellulose (crystalline cellulose), low-alkyl-substituted hydroxypropyl cellulose (L-HPC), and sodium carboxymethyl starch; more preferably, at least one selected from croscarmellose sodium and sodium starch glycolate; further preferably croscarmellose sodium or sodium starch glycolate.
[7] The oral solid formulation according to any one of aspects [3] to [3-2], wherein the glidant is at least one selected from, for example, magnesium stearate, calcium stearate, zinc stearate, sodium stearyl fumarate, a mixture of magnesium stearate and sodium lauryl sulfate, talc, carboxymethyl cellulose, stearic acid, sodium lauryl sulfate, carnauba wax, sucrose fatty acid ester, polyethylene glycol, glycerin, glyceryl monostearate, castor oil, and hydrogenated castor oil.
[7-1] In aspect [7], preferably, the glidant is at least one selected from the group consisting of magnesium stearate, calcium stearate, sodium stearyl fumarate, a mixture of magnesium stearate and sodium lauryl sulfate, talc, stearic acid, sodium lauryl sulfate and carnauba wax; more preferably at least one selected from magnesium stearate and sodium stearyl fumarate; further preferably magnesium stearate or sodium stearyl fumarate.
[8] The oral solid formulation according to any one of aspects [1] to [7-1], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 13.0% or more (excluding the oral solid formulation in which the coating agent is methacrylic acid copolymer S).
[8A] The oral solid formulation according to any one of aspects [1] to [7-1], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is, for example, about 10.0% or more, about 13.0% or more, about 15.0% or more, about 18.0% or more or about 20.0% or more (excluding the oral solid formulation in which the coating agent is methacrylic acid copolymer S).
[8B] The oral solid formulation according to any one of aspects [1] to [7-1], wherein when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 6 hours after the start of the dissolution test is, for example, about 10.0% or more, about 13.0% or more, or about 15.0% or more.
[8C] The oral solid formulation according to any one of aspects [1] to [7-1], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour or 6 hours after the start of the dissolution test is, for example, about 10.0% or more, about 13.0% or more, or about 15.0% or more.
[9] The oral solid formulation according to any one of aspects [1] to [8C], wherein, when a dissolution test is performed using Dissolution Test Solution 1 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 2.5% or less.
[9-1] In aspect [9], preferably, the dissolution rate is about 2.0% or less; more preferably about 1.0% or less; further preferably about 0.5% or less.
[10] The oral solid formulation according to any one of aspects [1] to [9-1], wherein the coating layer begins to dissolve in a solution with pH=about 5.0 to about 7.0, and the active ingredient in the formulation is thereby dissolved.
[10-1] In aspect [10], the pH at which the coating layer begins to dissolve is, for example, a range having an upper limit and/or a lower limit of pH=about 5.0, about 5.5, about 6.0, about 6.5, or about 7.0 (e.g., a range of about 5.0 to about 7.0, about 5.0 to about 6.5, about 5.0 to about 6.0, about 5.0 to about 5.5, about 5.5 to about 7.0, about 5.5 to about 6.5, about 5.5 to about 6.0, about 6.0 to about 7.0, about 6.0 to about 6.5, or about 6.5 to about 7.0), preferably pH=about 5.0 to about 7.0; more preferably pH=about 5.0, about 5.5, about 6.0, about 6.5, or about 7.0.
[11] An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
   (1) a core containing the following components (a) to (e):
      (a) the active ingredient;
      (b) at least one excipient selected from the group consisting of crystalline cellulose, granulated lactose, and crystalline cellulose;
      (c) at least one flow agent selected from the group consisting of light anhydrous silicic acid, talc, and magnesium aluminometasilicate;
      (d) at least one disintegrant selected from croscarmellose sodium and sodium starch glycolate; and
      (e) at least one glidant selected from magnesium stearate and sodium stearyl fumarate; and
   (2) a coating layer covering the core, the coating layer containing at least one coating agent selected from the group consisting of hypromellose phthalate, methacrylic acid copolymer, hypromellose acetate succinate, cellulose acetate phthalate, and carboxymethyl ethyl cellulose.
[11-X1] In aspect [11], preferably, the coating agent for coating the core is more preferably one selected from the group consisting of hypromellose phthalate, methacrylic acid copolymer, hypromellose acetate succinate, cellulose acetate phthalate, and carboxymethyl ethyl cellulose.
[11-X2] In aspect [11] or [11-X1], the methacrylic acid copolymer is, for example, methacrylic acid copolymer LD, methacrylic acid copolymer L, methacrylic acid copolymer S, dry methacrylic acid copolymer LD, etc., preferably, the methacrylic acid copolymer is, for example, at least one selected from the group consisting of methacrylic acid copolymer LD, methacrylic acid copolymer L, methacrylic acid copolymer S, and dry methacrylic acid copolymer LD; more preferably, one selected from the group consisting of methacrylic acid copolymer LD, methacrylic acid copolymer L, methacrylic acid copolymer S, and dry methacrylic acid copolymer LD.
[11-1] The oral solid formulation according to any one of aspects [11] to [11-X2], wherein the coating layer further contains triethyl citrate.
[11-2] The oral solid formulation according to any one of aspects [11] to [11-X2], wherein the coating layer further contains titanium dioxide or/and talc.
[11-3] The oral solid formulation according to any one of aspects [11] to [11-X2], wherein the coating layer further contains triethyl citrate, titanium dioxide and talc.
[11-4] The oral solid formulation according to any one of aspects [11] to [11-3], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 13.0% or more (excluding the oral solid formulation in which the coating agent is methacrylic acid copolymer S).
[11-4A] The oral solid formulation according to any one of aspects [11] to [11-3], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is, for example, about 10.0% or more, about 13.0% or more, about 15.0% or more, about 18.0% or more, or about 20.0% or more (excluding the oral solid formulation in which the coating agent is methacrylic acid copolymer S).
[11-4B] The oral solid formulation according to any one of aspects [11] to [11-3], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 6 hours after the start of the dissolution test is, for example, about 10.0% or more, about 13.0% or more, or about 15.0% or more.
[11-4C] The oral solid formulation according to any one of aspects [11] to [11-3], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour or 6 hours after the start of the dissolution test is, for example, about 10.0% or more, about 13.0% or more, or about 15.0% or more.
[11-5] The oral solid formulation according to any one of aspects [11] to [11-4C], wherein, when a dissolution test is performed using Dissolution Test Solution 1 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 2.5% or less.
[11-6] In aspect [11-5], preferably, the dissolution rate is about 2.0% or less; more preferably about 1.0% or less; further preferably about 0.5% or less.
[11-7] The oral solid formulation according to any one of aspects [11] to [11-6], wherein the coating layer begins to dissolve in a solution with pH=about 5.0 to about 7.0, and the active ingredient in the formulation is thereby dissolved.
[11-8] In aspect [11-7], the pH at which the coating layer begins to dissolve is, for example, a range having an upper limit and/or a lower limit of pH=about 5.0, about 5.5, about 6.0, about 6.5, or about 7.0 (e.g., a range of about 5.0 to about 7.0, about 5.0 to about 6.5, about 5.0 to about 6.0, about 5.0 to about 5.5, about 5.5 to about 7.0, about 5.5 to about 6.5, about 5.5 to about 6.0, about 6.0 to about 7.0, about 6.0 to about 6.5, or about 6.5 to about 7.0), preferably pH=about 5.0 to about 7.0; more preferably pH=about 5.0, about 5.5, about 6.0, about 6.5, or about 7.0.
[11A] An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
   (1) a core containing the following components (a) to (e):
      (a) the active ingredient;
      (b) at least one excipient selected from the group consisting of crystalline cellulose, granulated lactose, and crystalline cellulose;
      (c) at least one flow agent selected from the group consisting of light anhydrous silicic acid, talc, and magnesium aluminometasilicate;
      (d) at least one disintegrant selected from croscarmellose sodium and sodium starch glycolate; and
      (e) at least one glidant selected from magnesium stearate and sodium stearyl fumarate; and
   (2) a coating layer covering the core, the coating layer containing at least one coating agent in which the coating layer of the formulation begins to dissolve in a solution within the range of pH=about 5.0 to about 7.0.
[11A-1] In aspect [11A], the coating agent for coating the core has, for example, a range with an upper limit and/or a lower limit of pH=about 5.0, about 5.5, about 6.0, about 6.5, or about 7.0 (e.g., about 5.0 to about 7.0, about 5.0 to about 6.5, about 5.0 to about 6.0, about 5.0 to about 5.5, about 5.5 to about 7.0, about 5.5 to about 6.5, about 5.5 to about 6.0, about 6.0 to about 7.0, about 6.0 to about 6.5, or about 6.5 to about 7.0); preferably, it is a coating agent in which the coating layer of the formulation begins to dissolve in a solution within the range of pH=about 5.0 to about 7.0; more preferably, it is a coating agent in which the coating layer of the formulation begins to dissolve in a solution of pH=about 5.0, about 5.5, about 6.0, about 6.5, or about 7.0.
[11A-2] In aspect [11A] or [11A-1], the coating agent for coating the core is, for example, at least one coating agent selected from the group consisting of hypromellose phthalate, methacrylic acid copolymer, hypromellose acetate succinate, cellulose acetate phthalate, and carboxymethyl ethyl cellulose.
[11A-3] In aspect [11A-2], the coating agent for coating the core is preferably one selected from the group consisting of hypromellose phthalate, methacrylic acid copolymer, hypromellose acetate succinate, cellulose acetate phthalate, and carboxymethyl ethyl cellulose.
[11A-3A] In aspects [11A-1] to [11A-3], the methacrylic acid copolymer is, for example, methacrylic acid copolymer LD, methacrylic acid copolymer L, methacrylic acid copolymer S, dry methacrylic acid copolymer LD, etc., preferably, the methacrylic acid copolymer is, for example, at least one selected from the group consisting of methacrylic acid copolymer LD, methacrylic acid copolymer L, methacrylic acid copolymer S, and dry methacrylic acid copolymer LD; more preferably, one selected from the group consisting of methacrylic acid copolymer LD, methacrylic acid copolymer L, methacrylic acid copolymer S, and dry methacrylic acid copolymer LD.
[11A-4] The oral solid formulation according to any one of aspects [11A] to [11A-3A], wherein the coating layer further contains triethyl citrate.
[11A-5] The oral solid formulation according to any one of aspects [11A] to [11A-3], wherein the coating layer further contains titanium dioxide or/and talc.
[11A-6] The oral solid formulation according to any one of aspects [11A] to [11A-3A], wherein the coating layer further contains triethyl citrate, titanium dioxide and talc.
[11A-7] The oral solid formulation according to any one of aspects [11A] to [11A-6], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is 13.0% or more (excluding the oral solid formulation in which the coating agent is methacrylic acid copolymer S).
[11A-7A] The oral solid formulation according to any one of aspects [11A] to [11A-6], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is, for example, about 10.0% or more, about 13.0% or more, about 15.0% or more, about 18.0% or more, or about 20.0% or more (excluding the oral solid formulation in which the coating agent is methacrylic acid copolymer S).
[11A-7B] The oral solid formulation according to any one of aspects [11A] to [11A-6], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 6 hours after the start of the dissolution test is, for example, about 10.0% or more, about 13.0% or more, or 15.0% or more.
[11A-7C] The oral solid formulation according to any one of aspects [11A] to [11A-6], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour or 6 hours after the start of the dissolution test is, for example, about 10.0% or more, about 13.0% or more, or 15.0% or more.
[11A-8] The oral solid formulation according to any one of aspects [11A] to [11A-7C], wherein, when a dissolution test is performed using Dissolution Test Solution 1 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 2.5% or less.
[11A-9] In aspect [11A-8], preferably, the dissolution rate is about 2.0% or less; more preferably about 1.0% or less; further preferably about 0.5% or less.
[11B] An oral solid formulation comprising 1-((1R,2R)-2-hydroxy-4,4-dimethyl-1,2,3,4-tetrahydronaphthalen-1-yl)-3-(2-phenylpyridin-3-yl)urea or a pharmaceutically acceptable salt thereof, or a solvate thereof, as an active ingredient, wherein the formulation includes
   (1) a core containing the following components (a) and (b):
      (a) the active ingredient;
      (b) at least one additive selected from the group consisting of excipients, flow agents, disintegrants, and glidants; and
   (2) a coating layer covering the core, the coating layer containing at least one coating agent in which the coating layer of the formulation begins to dissolve in a solution within a range of pH=about 5.0 to about 7.0.
[11B-1] In aspect [11B], preferably, the excipient is at least one component selected from the group consisting of lactose (monohydrate, spray-dried monohydrate, anhydrous, etc.), granulated lactose, white sugar, mannitol (D-mannitol), crystalline cellulose, microcrystalline cellulose, silicic acid, starch, maize starch, potato starch, anhydrous calcium hydrogen phosphate, corn starch, pregelatinized starch and partially pregelatinized starch; more preferably at least one selected from the group consisting of crystalline cellulose, mannitol (D-mannitol) and granulated lactose; further preferably one selected from the group consisting of mannitol, crystalline cellulose, a mixture of mannitol and crystalline cellulose, and a mixture of crystalline cellulose and granulated lactose.
[11B-2] In aspect [11B], preferably, the flow agent is at least one selected from the group consisting of light anhydrous silicic acid, talc, and magnesium aluminometasilicate; more preferably one selected from the group consisting of light anhydrous silicic acid, a mixture of light anhydrous silicic acid and talc, talc, and magnesium aluminometasilicate.
[11B-3] In aspect [11B], preferably, the disintegrant is at least one selected from the group consisting of sodium carboxymethyl starch, carmellose (carboxymethyl cellulose, CMC), croscarmellose sodium, crospovidone, sodium starch glycolate, carboxymethyl cellulose calcium, polyvinylpyrrolidone (PVPP), microcrystalline cellulose (crystalline cellulose), low-alkyl-substituted hydroxypropyl cellulose (L-HPC), and sodium carboxymethyl starch; more preferably at least one selected from croscarmellose sodium and sodium starch glycolate; further preferably croscarmellose sodium or sodium starch glycolate.
[11B-4] In aspect [11B], preferably, the glidant is at least one selected from the group consisting of magnesium stearate, calcium stearate, sodium stearyl fumarate, a mixture of magnesium stearate and sodium lauryl sulfate, talc, stearic acid, sodium lauryl sulfate and carnauba wax; more preferably at least one selected from magnesium stearate and sodium stearyl fumarate; further preferably magnesium stearate or sodium stearyl fumarate.
[11B-5] In aspect [11B], the coating agent for coating the core has, for example, a range with an upper limit and/or a lower limit of pH=about 5.0, about 5.5, about 6.0, about 6.5, or about 7.0 (e.g., about 5.0 to about 7.0, about 5.0 to about 6.5, about 5.0 to about 6.0, about 5.0 to about 5.5, about 5.5 to about 7.0, about 5.5 to about 6.5, about 5.5 to about 6.0, about 6.0 to about 7.0, about 6.0 to about 6.5, or about 6.5 to about 7.0); preferably, it is a coating agent in which the coating layer of the formulation begins to dissolve in a solution within the range of pH=about 5.0 to about 7.0; more preferably, it is a coating agent in which the coating layer of the formulation begins to dissolve in a solution of pH=about 5.0, about 5.5, about 6.0, about 6.5, or about 7.0.
[11B-6] In aspects [11B] to [11B-5], the coating agent for coating the core is at least one coating agent selected from the group consisting of hypromellose phthalate, methacrylic acid copolymer, hypromellose acetate succinate, cellulose acetate phthalate, and carboxymethyl ethyl cellulose.
[11B-7] In aspect [11B-6], preferably, the coating agent for coating the core is one selected from the group consisting of hypromellose phthalate, methacrylic acid copolymer, hypromellose acetate succinate, cellulose acetate phthalate, and carboxymethyl ethyl cellulose.
[11B-7A] In aspect [11B-6] or [11B-7], the methacrylic acid copolymer is, for example, methacrylic acid copolymer LD, methacrylic acid copolymer L, methacrylic acid copolymer S, dry methacrylic acid copolymer LD, etc., preferably, the methacrylic acid copolymer is at least one selected from the group consisting of methacrylic acid copolymer LD, methacrylic acid copolymer L, methacrylic acid copolymer S, and dry methacrylic acid copolymer LD; more preferably, one selected from the group consisting of methacrylic acid copolymer LD, methacrylic acid copolymer L, methacrylic acid copolymer S, and dry methacrylic acid copolymer LD.
[11B-8] The oral solid formulation according to any one of aspects [11B] to [11B-7A], wherein the coating layer further contains triethyl citrate.
[11B-9] The oral solid formulation according to any one of aspects [11B] to [11B-7A], wherein the coating layer further contains titanium dioxide or/and talc.
[11B-10] The oral solid formulation according to any one of aspects [11B] to [11B-7A], wherein the coating layer further contains triethyl citrate, titanium dioxide and talc.
[11B-11] The oral solid formulation according to any one of aspects [11B] to [11B-10], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 13.0% or more (excluding the oral solid formulation in which the coating agent is methacrylic acid copolymer S).
[11B-11A] The oral solid formulation according to any one of aspects [11B] to [11B-10], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is, for example, about 10.0% or more, about 13.0% or more, about 15.0% or more, about 18.0% or more or about 20.0% or more (excluding the oral solid formulation in which the coating agent is methacrylic acid copolymer S).
[11B-11B] The oral solid formulation according to any one of aspects [11B] to [11B-10], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 6 hours after the start of the dissolution test is, for example, about 10.0% or more, about 13.0% or more, or about 15.0% or more.
[11B-11C] The oral solid formulation according to any one of aspects [11B] to [11B-10], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour or 6 hours after the start of the dissolution test is, for example, about 10.0% or more, about 13.0% or more, or about 15.0% or more.
[11B-12] The oral solid formulation according to any one of aspects [11B] to [11B-11C], wherein, when a dissolution test is performed using Dissolution Test Solution 1 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is about 2.5% or less.
[11B-13] In aspect [11B-12], preferably, the dissolution rate is about 2.0% or less; more preferably about 1.0% or less; further preferably about 0.5% or less.
[12] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein 12 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 100 nM or more.
[12A] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein 12 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 90 nM or more, about 95 nM or more, about 100 nM or more, about 105 nM or more, or about 110 nM or more.
[12-1] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 150 mg, or a pharmaceutically acceptable salt thereof in an amount equivalent to the amount, or a solvate thereof in an amount equivalent to the amount, wherein 12 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 100 nM or more.
[12-1A] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 150 mg, or a pharmaceutically acceptable salt thereof in an amount equivalent to the amount, or a solvate thereof in an amount equivalent to the amount, wherein 12 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 90 nM or more, about 95 nM or more, about 100 nM or more, about 105 nM or more, or about 110 nM or more.
[12-2] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein either 12 hours or 24 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 100 nM or more.
[12-2A] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein either 12 hours or 24 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 90 nM or more, about 95 nM or more, about 100 nM or more, about 105 nM or more, or about 110 nM or more.
[12-3] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 150 mg, or a pharmaceutically acceptable salt thereof in an amount equivalent to the amount, or a solvate thereof in an amount equivalent to the amount, wherein either 12 hours or 24 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 100 nM or more.
[12-3A] The oral solid formulation according to any one of aspects [1] to [11B-13], which contains 1-((1R,2R)-2-hydroxy-4,4-dimethyl-1,2,3,4-tetrahydronaphthalen-1-yl)-3-(2-phenylpyridin-3-yl)urea in an amount of 150 mg, or a pharmaceutically acceptable salt thereof in an amount equivalent to the amount, or a solvate thereof in an amount equivalent to the amount, wherein either 12 hours or 24 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes about 90 nM or more, about 95 nM or more, about 100 nM or more, about 105 nM or more, or about 110 nM or more.
[12-4] In aspects [12] to [12-3A], the administration subject is a human or non-human mammal (dog, cat, rat, mouse, monkey, cattle, horse, pig, sheep, etc.).
[13] The oral solid formulation according to any one of aspects [1] to [12-4], wherein the content of the coating agent for coating the core is, for example, in the range of about 1.0 to about 25.0% by mass, preferably in the range of about 2.0 to about 20.0% by mass, and more preferably in the range of about 3.5 to about 15.0% by mass or about 5.0 to about 15.0% by mass, relative to the total mass of the oral solid formulation.
[14] The oral solid formulation according to any one of aspects [1] to [13], wherein the content of the plasticizer that can be contained in the coating layer is, for example, in the range of 0.0 to about 10.0% by mass, preferably in the range of about 0.1 to about 5.0% by mass, and more preferably in the range of about 0.5 to about 3.0% by mass, relative to the total mass of the oral solid formulation.
[15] The oral solid formulation according to any one of aspects [1] to [14], wherein the content of the second coating agent that can be further contained in the coating layer is, for example, in the range of 0.0 to about 25.0% by mass, preferably in the range of about 1.0 to about 20.0% by mass, and more preferably in the range of about 1.0 to about 6.0% by mass, relative to the total mass of the oral solid formulation.
[16] The oral solid formulation according to any one of aspects [1] to [15], wherein the content of the excipient is, for example, in the range of about 20.0 to about 90.0% by mass, preferably in the range of about 30.0 to about 80.0% by mass, and more preferably in the range of about 40.0 to about 75.0% by mass, relative to the total mass of the oral solid formulation.
[17] The oral solid formulation according to any one of aspects [1] to [16], wherein the content of the flow agent is, for example, in the range of 0.0 to about 15.0% by mass, preferably in the range of about 0.1 to about 10.0% by weight, and more preferably in the range of about 0.5 to about 5.0% by mass, relative to the total mass of the oral solid formulation.
[18] The oral solid formulation according to any one of aspects [1] to [17], wherein the content of the disintegrant is, for example, in the range of 0.0 to about 30.0% by mass, preferably in the range of about 1.0 to about 20.0% by mass, and more preferably in the range of about 2.0 to about 8.0% by mass, relative to the total mass of the oral solid formulation.
[19] The oral solid formulation according to any one of aspects [1] to [18], wherein the content of the glidant is, for example, in the range of 0.0 to about 10.0% by mass, preferably in the range of about 0.1 to about 5.0% by mass, more preferably in the range of about 0.5 to about 3.0% by mass, relative to the total mass of the oral solid formulation.
[20] The oral solid formulation according to any one of aspects [1] to [19], wherein the release property of the active ingredient is intestinal quick-release.
[20-1] The oral solid formulation according to any one of aspects [1] to [19], wherein the release property of the active ingredient is gastric sustained-release or insoluble.
[20-2] The oral solid formulation according to any one of aspects [1] to [19], wherein the release property of the active ingredient is gastric sustained-release or insoluble and intestinal quick-release.
[21] The oral solid formulation according to any one of aspects [1] to [20-2], for use in prevention and/or treatment of a disease in which TrkA is involved.
[21-1] In aspect [21], the disease in which TrkA is involved is at least one selected from the group consisting of pain, cancer, inflammation/inflammatory diseases, allergic diseases, skin diseases, pruritus, neurodegenerative diseases, infectious diseases, Sjögren's syndrome, endometriosis, kidney diseases, osteoporosis, etc.
[21-2] In aspect [21-1], preferably, the disease in which TrkA is involved is at least one selected from pain (nociceptive pain represented by osteoarthritis, rheumatoid arthritis, fractures, interstitial cystitis, chronic pancreatitis, pain associated with prostatitis, chronic low back pain, diabetic peripheral neuropathic pain, postoperative pain, pelvic pain, cancer pain, etc., neuropathic pain, acute pain, chronic pain, inflammatory pain, etc.), and pruritus (systemic pruritus, localized pruritus, generalized pruritus, etc.).
[21-3] In [21], the disease in which TrkA is involved is at least one selected from the group consisting of osteoarthritic pain, joint pain, neuropathic pain, postoperative pain, low back pain, diabetic neuropathy, intraoperative pain, cancer pain, chemotherapyinduced pain, headache (including cluster headache, tension headache, migraine pain), trigeminal neuralgia, herpes zoster pain, postherpetic neuralgia, carpal tunnel syndrome, inflammatory pain, pain from rheumatoid arthritis, colitis, pain from interstitial cystitis, visceral pain, pain from kidney stones, pain from gallstones, sore throat, fibromyalgia, chronic pain syndrome, thalamic pain syndrome, pain from stroke, phantom limb pain, sunburn, radiculopathy, complex regional pain syndrome, HIV sensory neuropathy, central pain syndrome, multiple sclerosis pain, Parkinson's disease pain, spinal cord injury pain, dysmenorrhea, toothache, pain from bone metastasis, pain from endometriosis, pain from uterine fibroids, nociceptive pain, hyperalgesia, temporomandibular joint pain, neuroblastoma, ovarian cancer, endometrial cancer, glioblastoma multiforme, cervical cancer, pancreatic cancer, colon cancer, rectal cancer, prostate cancer, melanoma, myeloma, thyroid cancer, lung cancer (small cell lung cancer, non-small cell lung cancer), brain tumor, esophageal cancer, kidney cancer, bone tumor, hematological cancer (chronic myelogenous leukemia, acute lymphoblastic leukemia, Philadelphia chromosome-positive acute lymphoblastic leukemia (Ph+ALL), acute myeloid leukemia (AML), chronic lymphocytic leukemia (CML)), squamous cell carcinoma, glioma, gastrointestinal cancer, ovarian cancer, liver cancer, stomach cancer, bladder cancer, hepatocellular carcinoma, breast cancer, head and neck cancer, germ cell tumor, pediatric sarcoma, extranodal NK/T-cell lymphoma, nasal type, multiple myeloma, interstitial cystitis (IC), painful bladder syndrome (PBS), urinary incontinence, inflammatory cystitis, inflammatory bowel disease, ulcerative colitis, Crohn's disease, systemic lupus erythematosus, rheumatoid arthritis, joint swelling, asthma, atopic dermatitis, psoriasis, psoriatic arthritis, rhinitis, systemic pruritus, localized pruritus, generalized pruritus, multiple sclerosis, Parkinson's disease, Alzheimer's disease, Chagas disease, Sjögren's syndrome, endometriosis, diabetic nephropathy, renal fibrosis, chronic kidney disease, osteoporosis, etc.

The present invention provides an oral solid formulation containing compound (I) which, when administered to a subject, exhibits effects such as (1) enabling control of a rapid increase in the blood concentration of compound (I) and (2) sustaining the blood concentration of compound (I) for a prolonged period of time.

### Brief Description of Drawings

Fig. 1 shows data on the results of the dissolution test in Test Example 1.
Fig. 2 shows data on the results of the dissolution test in Test Example 2.
Fig. 3 shows data on the results of the dissolution test in Test Example 3.
Fig. 4 shows data on the results of the dissolution test in Test Example 4.
Fig. 5 shows data on the results of the dissolution test in Test Example 5.
Fig. 6 shows data on the results of the dissolution test in Test Example 6.
Fig. 7 shows data on the results of the dissolution test in Test Example 7.
Fig. 8 shows data on the results of the dissolution test in Test Example 8.
Fig. 9 is a graph showing the time course of plasma concentration of compound (I) when the oral solid formulation produced by the formulation prescriptions of (Comparative Example 1) was orally administered to beagle dogs.
Fig. 10 is a graph showing the time course of plasma concentration of compound (I) when the oral solid formulation produced by the formulation prescriptions of (Example 1-1) was orally administered to beagle dogs.
Fig. 11 is a graph showing the time course of plasma concentration of compound (I) when the oral solid formulation produced by the formulation prescriptions of (Example 1-2) was orally administered to beagle dogs.
Fig. 12 is a graph showing the time course of plasma concentration of compound (I) when the oral solid formulation produced by the formulation prescriptions of (Example 2-1) was orally administered to beagle dogs.
Fig. 13 is a graph showing the time course of plasma concentration of compound (I) when the oral solid formulation produced by the formulation prescriptions of (Example 2-2) was orally administered to beagle dogs.
Fig. 14 is a graph showing the time course of plasma concentration of compound (I) when the oral solid formulation produced by the formulation prescriptions of (Example 2-3) was orally administered to beagle dogs.
Fig. 15 is a graph showing the time course of plasma concentration of compound (I) when the oral solid formulation produced by the formulation prescriptions of (Example 2-4) was orally administered to beagle dogs.
Fig. 16 is a graph showing the time course of plasma concentration of compound (I) when the oral solid formulation produced by the formulation prescriptions of (Example 2-5) was orally administered to beagle dogs.
Fig. 17 shows data on the results of the dissolution test in Test Example 9.
Fig. 18 shows data on the results of the dissolution test in Test Example 10.
Fig. 19 is a graph showing the time course of plasma concentration of compound (I) when the oral solid formulation produced by the formulation prescriptions of (Example 5-1) was orally administered to beagle dogs.
Fig. 20 is a graph showing the time course of plasma concentration of compound (I) when the oral solid formulation produced by the formulation prescriptions of (Example 5-2-1) was orally administered to beagle dogs.
Fig. 21 is a graph showing the time course of plasma concentration of compound (I) when the oral solid formulation produced by the formulation prescriptions of (Example 5-3) was orally administered to beagle dogs.
Fig. 22 is a graph showing the time course of plasma concentration of compound (I) when the oral solid formulation produced by the formulation prescriptions of (Example 5-4) was orally administered to beagle dogs.

### Description of Embodiments

The present invention relates to an oral solid formulation containing 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient.

Hereinafter, embodiments of the present invention will be described in detail. However, the present invention is not limited to the following embodiments, and can be implemented in any form without departing from the spirit of the present invention. Furthermore, the preferred and more preferred embodiments exemplified below may be used in combination with each other as appropriate, regardless of expressions such as "for example," "preferred," and "more preferred." In addition, the description of numerical ranges is an example, and ranges in which the upper and lower limits of each range and numerical values of the examples are appropriately combined can be preferably used. Further, the terms "contains" or "includes" are to be interpreted as inclusive, open-ended transitional phrases and not as limiting, closed-ended transitional phrases. The terms "contains" or "includes" may be read as "consisting essentially of" or "consisting of."

In the present invention, the "core" refers to a solid, preferably a tablet or its center, in which the active ingredient 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, and pharmaceutically acceptable additives are compression-molded together. The shape and size of the core are not particularly limited, but, for example, it is preferable that the core surface has a curved surface that can be coated with a coating agent. Furthermore, the shape of the solid formulation having a coating layer obtained by coating the core with a coating agent may include, for example, a shape that is easy to handle as a formulation and easy for a subject to take, for example, a circular shape, a triangular shape, an elliptical shape, etc.

In the present invention, the method for producing a core containing 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient is not particularly limited, but it is possible to select production methods generally used for the production of formulation cores and tablets, such as a direct compression method, a dry granulation method, a wet granulation method, and a fluidized bed granulation method.

Pharmaceutically acceptable additives are not particularly limited as long as they are used for formulation, but it is possible to use additives described in reference books on pharmaceutical research, such as "Japanese Pharmaceutical Excipients Directory 2021, edited by Japan Pharmaceutical Excipients Council, February 2021" (the contents of which are incorporated herein by reference). For example, additives such as excipients, disintegrants, flow agents, glidants, binders, stabilizers, coloring agents, and the like can be used as components used to maintain functions such as formulation manufacturability, appropriate hardness, disintegration, and dissolution of the formulation.

The additives contained in the core of the present invention are at least one selected from the group consisting of excipients, flow agents, disintegrants, and glidants, preferably excipients, flow agents, disintegrants, and glidants. The core of the present invention may further contain additives such as binders, stabilizers, surfactants, pH adjusters, solubilizers, antioxidants, adsorbents, corrigents, sweeteners, coloring agents, flavoring agents, and the like.

In the present invention, the "coating layer" refers to a coating agent (including a second coating agent) described later (for example, hypromellose phthalate, methacrylic acid copolymer, hypromellose acetate succinate, aminoalkyl methacrylate copolymer, cellulose acetate phthalate, carboxymethyl ethyl cellulose, hydroxypropyl cellulose, hydroxypropyl methylcellulose, titanium dioxide, talc, etc.) used to coat the core, and means a layer laminated or coated on the surface of the core.

The surface of the core of the present invention is entirely covered with the coating layer. The oral solid formulation of the present invention is, for example, a solution in the range of pH=about 5.0 to about 7.0, or a range having an upper limit and/or a lower limit of pH=about 5.0, about 5.5, about 6.0, about 6.5, or about 7.0 (e.g., about 5.0 to about 7.0, about 5.0 to about 6.5, about 5.0 to about 6.0, about 5.0 to about 5.5, about 5.5 to about 7.0, about 5.5 to about 6.5, about 5.5 to about 6.0, about 6.0 to about 7.0, about 6.0 to about 6.5, or about 6.5 to about 7.0), preferably, it has a property that it dissolves when it comes into contact with a solution in a range of pH=about 5.0 to about 7.0, more preferably a solution of pH=about 5.0, about 5.5, about 6.0, about 6.5, or about 7.0, and releases the active ingredient contained in the core.

The coating layer may further contain additives such as plasticizers, excipients, binders, disintegrants, lubricants, surfactants, pH adjusters, solubilizers, stabilizers, antioxidants, adsorbents, corrigents, sweeteners, coloring agents, flavoring agents, flow agents, and glidants.

Additives (for example, binders, stabilizers, surfactants, pH adjusters, solubilizers, antioxidants, adsorbents, corrigents, sweeteners, coloring agents, flavoring agents, etc.) that can be additionally contained in the core or the coating layer can be appropriately selected from additives described in reference books on pharmaceutical research, for example, "Japanese Pharmaceutical Excipients Directory 2021, edited by Japan Pharmaceutical Excipients Council, February 2021." The additives exemplified here that can be additionally contained in the core or the coating layer can be added to both the core and the coating layer. Furthermore, one compound may play multiple roles (for example, excipient and binder, etc.).

In the present invention, the "solution" includes liquids such as aqueous solutions and body fluids. A solution with a pH of about 1 to about 2 corresponds to a solution with a pH of gastric juice, and a solution with a pH of about 4 to about 8 corresponds to a solution with a pH of intestinal juice.

Coating can be performed using a coating method generally used in the technical field. Specifically, methods such as a pan coating method, a fluidized bed coating method, a ventilated dry pan coating method, a tumbling coating method, a spray coating method, and the like can be mentioned. Coating can be performed using a coating apparatus according to each coating method.

**In** coating, it is also possible to coat the surface of the core using a coating liquid in which additives such as coloring agents, corrigents, sweeteners, flavoring agents, and the like are blended as necessary with the coating agents mentioned above.

The proportion of the solvent (water, organic solvent, etc.) used in producing the coating agent is not particularly limited, but can be used in any proportion. The type of the organic solvent is not particularly limited, and, for example, alcohol solvents (for example, methanol, ethanol, isopropyl alcohol, etc.), ketones (for example, acetone, etc.), halogenated hydrocarbons (for example, chloroform, dichloromethane, etc.), and the like are used.

**In** the present invention, "oral solid formulation" means a solid formulation type for oral administration. Examples of the oral solid formulation include tablets, powders, granules, capsules, troches, pills, and the like. The oral solid formulation of the present invention is preferably a tablet. The tablet is not particularly limited, and examples thereof include tablets of types such as orally disintegrating tablets, chewable tablets, effervescent tablets, dispersible tablets, soluble tablets, sugar-coated tablets, and coated tablets. The oral solid formulation of the present invention is preferably a coated tablet.

The oral solid formulation of the present invention is prepared by blending 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient with a pharmaceutical carrier (additives such as excipients, disintegrants, flow agents, and glidants), and performing granulation, tableting, and coating with a coating agent (which may contain a plasticizer, etc.); it is preferably an oral solid formulation (tablet) having a uniform content of the active ingredient (for example, 5 mg, 10 mg, 20 mg, 30 mg, 50 mg, 100 mg, 150 mg, etc.).

1-((1R,2R)-2-hydroxy-4,4-dimethyl-1,2,3,4-tetrahydronaphthalen-1-yl)-3-(2-phenylpyridin-3-yl)urea is described in WO 2018/199166 pamphlet or WO 2019/189717 pamphlet, and can be produced by the production methods described in the pamphlets.

The pharmaceutically acceptable salts of 1-((1R,2R)-2-hydroxy-4,4-dimethyl-1,2,3,4-tetrahydronaphthalen-1-yl)-3-(2-phenylpyridin-3-yl)urea (compound (I)) are not particularly limited as long as they are pharmaceutically acceptable salts, and examples include mineral acids such as hydrochloric acid, hydrobromic acid, hydroiodic acid, sulfuric acid, nitric acid, and phosphoric acid; aliphatic monocarboxylic acids such as formic acid, acetic acid, propionic acid, butyric acid, valeric acid, enanthic acid, capric acid, myristic acid, palmitic acid, stearic acid, lactic acid, sorbic acid, and mandelic acid; aromatic monocarboxylic acids such as benzoic acid and salicylic acid; aliphatic dicarboxylic acids such as oxalic acid, malonic acid, succinic acid, fumaric acid, maleic acid, malic acid, and tartaric acid; organic carboxylic acids such as aliphatic tricarboxylic acids including citric acid; acid addition salts with organic sulfonic acids such as aliphatic sulfonic acids including methanesulfonic acid, ethanesulfonic acid, and 2-hydroxyethanesulfonic acid and aromatic sulfonic acids including benzenesulfonic acid and p-toluenesulfonic acid, inorganic base addition salts with metals such as alkali metals or alkaline earth metals including sodium, potassium, magnesium, and calcium, and organic base addition salts such as methylamine, ethylamine, ethanolamine, pyridine, lysine, arginine, and ornithine. These salts can be obtained by conventional methods, for example, by mixing an equivalent amount of compound (I) with a solution containing a desired acid or base, and collecting the desired salt by filtration or by distilling off the solvent.

In the present invention, "solvate" refers to a molecular complex comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt of 1-((1R,2R)-2-hydroxy-4,4-dimethyl-1,2,3,4-tetrahydronaphthalen-1-yl)-3-(2-phenylpyridin-3-yl)urea, and one or more pharmaceutically acceptable solvent molecules (for example, water, ethanol, etc.). When the solvent molecule is water, it is particularly called "hydrate."

The content of 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, used in the present invention is, for example, in a range of about 5.0 to about 70.0% by mass, preferably in a range of about 10.0 to about 50.0% by mass, and more preferably in a range of about 10.0 to about 30.0% by mass, relative to the total mass of the oral solid formulation.

In the present invention, the "excipient" is an additive described in reference books on pharmaceutical research, for example, "Japanese Pharmaceutical Excipients Directory 2021, edited by Japan Pharmaceutical Excipients Council, February 2021," and is a component added for the purpose of obtaining a certain size and concentration in the formulation of tablets and the like. Examples of excipients include, but are not limited to, glucose, lactose (monohydrate, spray-dried monohydrate, anhydrous, etc.), granulated lactose, sucrose, white sugar, mannitol (D-mannitol), mannite, xylitol, sorbitol (D-sorbitol), crystalline cellulose, microcrystalline cellulose, silicic acid, starch, maize starch, potato starch, dicalcium phosphate dihydrate, isomalt, anhydrous calcium phosphate, anhydrous calcium hydrogen phosphate, corn starch, pregelatinized starch, partially pregelatinized starch, light anhydrous silicic acid, titanium dioxide, and mixtures thereof. Preferred excipients are at least one selected from the group consisting of lactose (monohydrate, spray-dried monohydrate, anhydrous, etc.), granulated lactose, white sugar, mannitol (D-mannitol), crystalline cellulose, microcrystalline cellulose, silicic acid, starch, maize starch, potato starch, anhydrous calcium hydrogen phosphate, corn starch, pregelatinized starch and partially pregelatinized starch; more preferably at least one selected from the group consisting of mannitol, crystalline cellulose and granulated lactose; and even more preferably one selected from the group consisting of mannitol, crystalline cellulose, a mixture of mannitol and crystalline cellulose, and a mixture of crystalline cellulose and granulated lactose.

The content of the excipient used in the present invention is, for example, in a range of about 20.0 to about 90.0% by mass, preferably in a range of about 30.0 to about 80.0% by mass, and more preferably in a range of about 40.0 to about 75.0% by mass, relative to the total mass of the oral solid formulation.

In the present invention, the "disintegrant" is an additive described in reference books on pharmaceutical research, for example, "Japanese Pharmaceutical Excipients Directory 2021, edited by Japan Pharmaceutical Excipients Council, February 2021," and is a component added for the purpose of disintegrating a formulation (tablet) by absorbing moisture in the body and swelling, etc., to facilitate the release of the active ingredient. Examples of disintegrants include, but are not limited to, starches (maize starch, potato starch, starch, pregelatinized starch, partially pregelatinized starch), sodium carboxymethyl starch, carmellose (carboxymethyl cellulose, CMC), carmellose calcium, croscarmellose sodium, crospovidone, sodium starch glycolate, sodium carboxymethyl cellulose, carboxymethyl cellulose calcium, polyvinylpyrrolidone (PVPP), methylcellulose, microcrystalline cellulose (crystalline cellulose), low-alkyl-substituted hydroxypropyl cellulose (L-HPC), sodium alginate, corn starch, sodium carboxymethyl starch, and mixtures thereof. Preferred disintegrants are at least one selected from the group consisting of sodium carboxymethyl starch, carmellose (carboxymethyl cellulose, CMC), croscarmellose sodium, crospovidone, sodium starch glycolate, carboxymethyl cellulose calcium, polyvinylpyrrolidone (PVPP), microcrystalline cellulose (crystalline cellulose), low-alkyl-substituted hydroxypropyl cellulose (L-HPC), and sodium carboxymethyl starch; more preferably, at least one selected from the group consisting of croscarmellose sodium and sodium starch glycolate; even more preferably croscarmellose sodium or sodium starch glycolate.

The content of the disintegrant used in the present invention is, for example, in a range of 0.0 to about 30.0% by mass, preferably in a range of about 1.0 to about 20.0% by mass, and more preferably in a range of about 2.0 to about 8.0% by mass, relative to the total mass of the oral solid formulation.

In the present invention, the "flow agent" is an additive described in reference books on pharmaceutical research, for example, "Japanese Pharmaceutical Excipients Directory 2021, edited by Japan Pharmaceutical Excipients Council, February 2021," and is a component added for the purpose of improving the flowability of the powder before tableting. Examples of flow agents include, but are not limited to, silicon dioxide (light anhydrous silicic acid, silica gel), talc, hydrous silicon dioxide, magnesium aluminometasilicate, granulated calcium hydrogen phosphate, and mixtures thereof. Preferred flow agents are at least one selected from the group consisting of light anhydrous silicic acid, talc, and magnesium aluminometasilicate; more preferably, one selected from the group consisting of light anhydrous silicic acid, a mixture of light anhydrous silicic acid and talc, talc, and magnesium aluminometasilicate.

The content of the flow agent used in the present invention is, for example, in a range of 0.0 to about 15.0% by mass, preferably in a range of about 0.1 to about 10.0% by weight, and more preferably in a range of about 0.5 to about 5.0% by mass, relative to the total mass of the oral solid formulation.

In the present invention, "glidant" is an additive described in reference books on pharmaceutical research, for example, "Japanese Pharmaceutical Excipients Directory 2021, edited by Japan Pharmaceutical Excipients Council, February 2021," and is a component suitably added for the purpose of eliminating insufficient hardness (core defects) of the core caused by adhesion of the active ingredient to the compression device, compression force, etc., in the step of producing the core by tableting. Examples of glidants include, but are not limited to, magnesium stearate, calcium stearate, zinc stearate, sodium stearyl fumarate, a mixture of magnesium stearate and sodium lauryl sulfate, talc, carboxymethyl cellulose, stearic acid, sodium lauryl sulfate, carnauba wax, sucrose fatty acid ester, polyethylene glycol, glycerin, glyceryl monostearate, castor oil, hydrogenated castor oil, and mixtures thereof. Preferred glidants are at least one selected from the group consisting of magnesium stearate, calcium stearate, sodium stearyl fumarate, a mixture of magnesium stearate and sodium lauryl sulfate, talc, stearic acid, sodium lauryl sulfate and carnauba wax; more preferably at least one selected from the group consisting of magnesium stearate and sodium stearyl fumarate; further preferably magnesium stearate or sodium stearyl fumarate.

The content of the glidant used in the present invention is, for example, in a range of 0.0 to about 10.0% by mass, preferably in a range of about 0.1 to about 5.0% by mass, more preferably in a range of about 0.5 to about 3.0% by mass, relative to the total mass of the oral solid formulation.

In the present invention, the "plasticizer" is an additive described in reference books on pharmaceutical research, for example, "Japanese Pharmaceutical Excipients Directory 2021, edited by Japan Pharmaceutical Excipients Council, February 2021," and is a component added for the purpose of enabling the active ingredient to be released from the formulation at a constant rate by interacting with the polymer used to allow the polymer to take a flexible structure. Examples of plasticizers include, but are not limited to, triethyl citrate, tributyl citrate, macrogol (polyethylene glycol), propylene glycol, sodium lauryl sulfate in ethanol, triacetin, polysorbate 80, and mixtures thereof. Preferred plasticizers are at least one selected from the group consisting of triethyl citrate, macrogol (polyethylene glycol), propylene glycol, triacetin, and polysorbate 80; more preferably triethyl citrate.

The content of the plasticizer used in the present invention is, for example, in a range of 0.0 to about 10.0% by mass, preferably in a range of about 0.1 to about 5.0% by mass, more preferably in a range of about 0.5 to about 3.0% by mass, relative to the total mass of the oral solid formulation.

The "coating agent" is an additive described in reference books on pharmaceutical research, for example, "Japanese Pharmaceutical Excipients Directory 2021, edited by Japan Pharmaceutical Excipients Council, February 2021," and is a component that can be added for the purpose of coating the surface of a solid formulation (solid preparation). The coating agent is not particularly limited, but a fat-soluble coating is preferable, and examples thereof include hypromellose phthalate, methacrylic acid copolymer, hypromellose acetate succinate, aminoalkyl methacrylate copolymer, cellulose acetate phthalate, carboxymethyl ethyl cellulose, hydroxypropyl cellulose, hydroxypropyl methylcellulose, ethyl cellulose, titanium dioxide, talc, and mixtures thereof.

In the present invention, examples of the coating agent that can be contained in the coating layer include hypromellose phthalate, methacrylic acid copolymer, hypromellose acetate succinate, cellulose acetate phthalate, carboxymethyl ethyl cellulose, hydroxypropyl cellulose, hydroxypropyl methylcellulose, and mixtures thereof. Preferred coating agents contained in the coating layer are at least one selected from the group consisting of hypromellose phthalate, methacrylic acid copolymer, hypromellose acetate succinate, cellulose acetate phthalate, and carboxymethyl ethyl cellulose; more preferably, one selected from the group consisting of hypromellose phthalate, methacrylic acid copolymer, hypromellose acetate succinate, cellulose acetate phthalate, and carboxymethyl ethyl cellulose.

Examples of the second coating agent that can be contained in the coating layer include titanium dioxide, talc, crystalline cellulose, calcium carbonate, shellac, and mixtures thereof. Preferred second coating agents are at least one selected from the group consisting of titanium dioxide, talc, crystalline cellulose, calcium carbonate, and shellac; more preferably, titanium dioxide and/or talc.

The content of the coating agent contained in the coating layer used in the present invention is, for example, in a range of about 1.0 to about 25.0% by mass, preferably in a range of about 2.0 to about 20.0% by mass, and more preferably in a range of about 3.5 to about 15.0% by mass.

The content of the second coating agent used in the present invention is, for example, in a range of 0.0 to about 25.0% by mass, preferably in a range of about 1.0 to about 20.0% by mass, relative to the total mass of the oral solid formulation.

In the present invention, an oral solid formulation prepared using one selected from the group consisting of hypromellose phthalate, methacrylic acid copolymer, hypromellose acetate succinate, cellulose acetate phthalate, and carboxymethyl ethyl cellulose as the coating agent contained in the coating layer is a formulation that is substantially insoluble in an acidic region but at least partially soluble in a weakly acidic to basic region. In the present invention, the acidic region, unless otherwise specified, means a pH of about 0.5 to about 4.5, preferably about 1.0 to about 2.0, and the weakly acidic to basic region, unless otherwise specified, means a pH of about 5.0 to 9.0, preferably about 5.0 to about 6.5, or about 5.0 to about 7.0.

In the present invention, the pH of a solution at which the coating layer of an oral solid formulation begins to dissolve and the active ingredient in the formulation is dissolved may also be referred to as "dissolution pH." The dissolution pH may be, for example, in a range of pH=about 4.0 to about 8.0 when mimicking the pH of intestinal fluid.

In the present invention, the dissolution pH of the oral solid formulation is, for example, in a range of pH=about 5.0 to about 7.0, about 5.0 to about 6.5, about 5.0 to about 6.0, about 5.0 to about 5.5, about 5.5 to about 7.0, about 5.5 to about 6.5, about 5.5 to about 6.0, about 6.0 to about 7.0, about 6.0 to about 6.5, about 6.5 to about 7.0. A method for setting the dissolution pH to a range of, for example, about 5.0 to about 7.0, etc., includes using a commercially available coating agent. An oral solid formulation capable of controlling the dissolution pH can be prepared by using, for example, those listed in Table 1 as a coating agent contained in the coating layer, either alone or in combination.

**Table 1**

| Coating Agent (Component Name) | Dissolution pH Range | Product Name: Grade (Manufacturer) | Catalog-listed Dissolution pH |
|---|---|---|---|
| Hypromellose Phthalate | About 5.0 to about 5.5 | HPMCP: HP-50 (Shin-Etsu Chemical Co., Ltd.) | 5.0 or more |
| | | HPMCP: HP-55: Indicated Viscosity (40 mPa·s) | 5.5 or more |
| | | HPMCP: HP-55S: Indicated Viscosity (170 mPa·s) | |
| | | (Shin-Etsu Chemical Co., Ltd.) | |
| Methacrylic Acid Copolymer | About 5.5 to about 7.0 | EUDRAGIT L30D-55: Aqueous Dispersion (30%) | 5.5 or more |
| | | EUDRAGIT L100-55: Powder (Evonik Japan Co., Ltd.) | |
| | | EUDRAGIT L100 (Evonik Japan Co., Ltd.) | 6.0 or more |
| | | EUDRAGIT S100 (Evonik Japan Co., Ltd.) | 7.0 or more |
| Hypromellose Acetate Succinate | About 5.5 to about 6.5 | HPMCAS: AS-LG: Granular Product | 5.5 or more |
| | | HPMCAS: AS-LF: Fine Powder Product (Shin-Etsu Chemical Co., Ltd.) | |
| | | HPMCAS: AS-MG: Granular Product | 6.0 or more |
| | | HPMCAS: AS-MF: Fine Powder | |
| | | Product (Shin-Etsu Chemical Co., Ltd.) | |
| | | HPMCAS: AS-HG: Granular Product | 6.5 or more |
| | | HPMCAS: AS-HF: Fine Powder Product (Shin-Etsu Chemical Co., Ltd.) | |
| Aminoalkyl Methacrylate Copolymer | About 5.0 or less | EUDRAGIT E100 | 5.0 or less |
| | | EUDRAGIT EPO (Evonik Japan Co., Ltd.) | |
| Cellulose Acetate Phthalate | About 6.0 or more | Cellacefate (Eastman Chemical Japan) | 6.0 or more |
| Carboxymethyl Ethyl Cellulose | About 6.0 or more | CMEC (Freund Corporation) | 6.0 or more |

It is also possible to combine a plurality of coating agents to adjust the dissolution pH. The method for adjusting the dissolution pH is not limited to these.

Methacrylic acid copolymers include methacrylic acid copolymer LD (for example, EUDRAGIT L30D-55 (molecular weight: up to 320,000 g/mol), etc.), methacrylic acid copolymer L (for example, EUDRAGIT L100 (molecular weight: up to 125,000 g/mol), etc.), and methacrylic acid copolymer S (for example, EUDRAGIT S100 (molecular weight: up to 125,000 g/mol), etc.), as well as dry methacrylic acid copolymer LD (for example, EUDRAGIT L100-55 (molecular weight: up to 320,000 g/mol), etc.) obtained by drying methacrylic acid copolymer LD.

Here, methacrylic acid copolymer LD is a copolymer of methacrylic acid and ethyl acrylate (ratio 1:1) (the following structural formula): 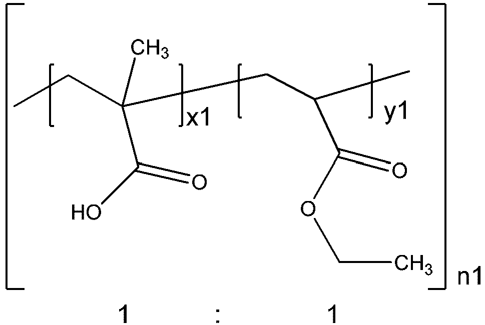 [wherein n1, x1, and y1 are arbitrary numbers determined according to the molecular weight], and usually methacrylic acid copolymer LD has the form of an emulsion obtained by copolymerizing methacrylic acid and ethyl acrylate in an aqueous solution containing polysorbate 80 and sodium lauryl sulfate. It usually dissolves in a solution with a pH of 5.5 or higher.

Methacrylic acid copolymer L is a copolymer of methacrylic acid and methyl methacrylate (ratio 1:1) (the following structural formula): 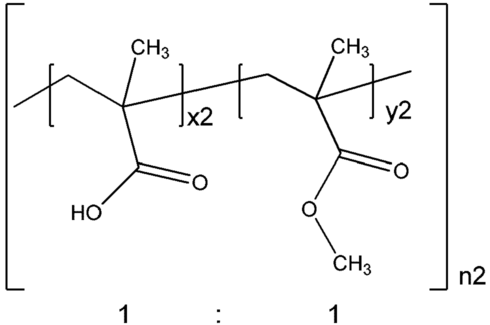 [wherein n2, x2, and y2 are arbitrary numbers determined according to the molecular weight], and has a powder form that usually dissolves in a solution with a pH of 6.0 or higher.

Methacrylic acid copolymer S is a copolymer of methacrylic acid and methyl methacrylate (ratio 1:2) (the following structural formula): 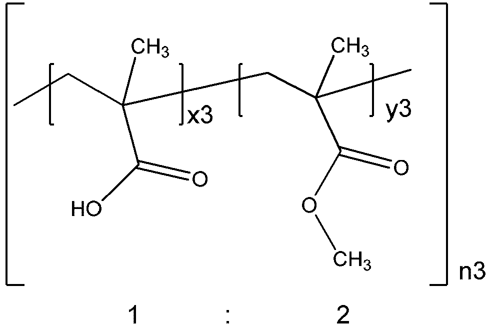 [wherein n3, x3, and y3 are arbitrary numbers determined according to the molecular weight], and has a powder form that usually dissolves in a solution with a pH of 7.0 or higher.

It is also possible to appropriately adjust the amount of the coating agent to be used. For example, the amounts of the coating agent (dry methacrylic acid copolymer LD) in the tablets (oral solid formulations) prepared in Examples 3-1 and 3-2 described later are 2 times and 3 times, respectively, the amount of the coating agent used in Example 2-1. For example, the amount of the coating agent (methacrylic acid copolymer L) in the tablet (oral solid formulation) prepared in Example 5-2-2 described later is about 0.7 times the amount of the coating agent used in Example 5-2-1.

In the present invention, the dissolution rate of the active ingredient contained in the oral solid formulation can be measured, for example, by a dissolution test method such as a rotating basket method of a dissolution test method described in Japanese Pharmacopoeia, Eighteenth Edition, or a paddle method of the same method.

In the present invention, the in vitro dissolution speed (dissolution rate) of the active ingredient contained in the oral solid formulation can be measured, for example, based on a dissolution test method described in Japanese Pharmacopoeia, Eighteenth Edition. This test method measures the time and ratio of dissolution of the active ingredient in a test solution in vitro. Examples of the test solution used for measurement include Dissolution Test Solution 1 (a liquid with a pH of about 1.2 simulating gastric juice, 2.0 g of sodium chloride dissolved in 7.0 mL of hydrochloric acid and water to make 1000 mL, also called JP1 solution) or Dissolution Test Solution 2 (a liquid with a pH of about 6.8 simulating intestinal fluid, obtained by adding 1 volume of water to 1 volume of phosphate buffer with a pH of 6.8, also called JP2 solution), but are not limited thereto. Examples of the measurement method include a rotating basket method of a dissolution test method described in Japanese Pharmacopoeia, Eighteenth Edition, and a paddle method of the same method.

In the present invention, when the in vitro dissolution speed of the active ingredient contained in the oral solid formulation is measured by the method described in the test examples below using Dissolution Test Solution 1 of Japanese Pharmacopoeia in accordance with the paddle method of the dissolution test method described in Japanese Pharmacopoeia, Eighteenth Edition, the dissolution rate of the active ingredient (compound (I)) 1 hour after is, for example, about 2.5% or less. When measured by the method described in the test examples below using Dissolution Test Solution 1 of Japanese Pharmacopoeia in accordance with the paddle method of the dissolution test method described in Japanese Pharmacopoeia, Eighteenth Edition, the dissolution rate of the active ingredient (compound (I)) 1 hour after the start of the dissolution test is preferably about 2.0% or less, more preferably about 1.0% or less, and even more preferably about 0.5% or less.

In the present invention, when the in vitro dissolution speed of the active ingredient contained in the oral solid formulation is measured by the method described in the test examples below using Dissolution Test Solution 2 of Japanese Pharmacopoeia in accordance with the paddle method of the dissolution test method described in Japanese Pharmacopoeia, Eighteenth Edition, the dissolution rate of the active ingredient (compound (I)) 1 hour after the start of the dissolution test is, for example, about 10.0% or more, about 13.0% or more, about 15.0% or more, about 18.0% or more, about 20.0% or more, and the like.

In the present invention, when the in vitro dissolution speed of the active ingredient contained in the oral solid formulation is measured by the method described in the test examples below using Dissolution Test Solution 2 of Japanese Pharmacopoeia in accordance with the paddle method of the dissolution test method described in Japanese Pharmacopoeia, Eighteenth Edition, the dissolution rate of the active ingredient (compound (I)) 6 hours after the start of the dissolution test may be, for example, about 10.0% or more, about 13.0% or more, or about 15.0% or more.

In the present invention, "Dissolution Test Solution 1 of Japanese Pharmacopoeia" may also be referred to as "Dissolution Test Solution 1." Furthermore, "Dissolution Test Solution 2 of Japanese Pharmacopoeia" may also be referred to as "Dissolution Test Solution 2."

When the pH in the in vitro described above is applied to the in vivo, it is known that the pH in the digestive tract is in a range of about 1 to about 2 in the stomach, in a range of about 4 to about 7 in the small intestine, and in a range of about 7 to about 8 in the large intestine, and the pH increases as it progresses to the lower digestive tract. Therefore, when a dissolution test is performed with Dissolution Test Solution 1 in accordance with the dissolution test method described in Japanese Pharmacopoeia, Eighteenth Edition, it corresponds to measuring the dissolution rate of the active ingredient contained in the oral solid formulation in a test solution simulating the fasting stomach; when a dissolution test is performed with Dissolution Test Solution 2 in accordance with the dissolution test method described in Japanese Pharmacopoeia, Eighteenth Edition, it corresponds to measuring the dissolution rate of the active ingredient contained in the oral solid formulation in a test solution simulating the intestine.

In the present invention, "release property" means the amount of release, release rate, etc., of the active ingredient contained in the oral solid formulation. The release rate means the amount of the active ingredient released from the formulation within a certain period of time.

In the present invention, "intestinal quick-release" means that when the dissolution rate of the oral solid formulation is tested with Dissolution Test Solution 2 in accordance with a dissolution test method described in Japanese Pharmacopoeia, Eighteenth Edition, the active ingredient (compound (I)) contained in the formulation shows a dissolution rate of about 13.0% or more of the total mass of the active ingredient (compound (I)) originally contained in the formulation 1 hour after the start of the dissolution test.

In the present invention, "gastric sustained-release or insoluble" means that when a dissolution test is performed with Dissolution Test Solution 1 in accordance with a dissolution test method described in Japanese Pharmacopoeia, Eighteenth Edition, the active ingredient (compound (I)) contained in the formulation shows a dissolution rate of about 2.5% or less of the total mass of the active ingredient (compound (I)) originally contained in the formulation 1 hour after the start of the dissolution test. Particularly, when the dissolution rate is about 0.5% or less, it becomes insoluble.

In the present invention, when "about" is described, it may also include values up to ±20%, preferably up to ±10% of the numerical value.

In the present invention, examples of diseases in which TrkA is involved include diseases such as pain, cancer, inflammation/inflammatory diseases, allergic diseases, skin diseases, pruritus, neurodegenerative diseases, infectious diseases, Sjögren's syndrome, endometriosis, kidney diseases, and osteoporosis.

More specifically, osteoarthritic pain, joint pain, neuropathic pain, postoperative pain, low back pain, diabetic neuropathy, intraoperative pain, cancer pain, chemotherapyinduced pain, headache (including cluster headache, tension headache, migraine pain), trigeminal neuralgia, herpes zoster pain, postherpetic neuralgia, carpal tunnel syndrome, inflammatory pain, pain from rheumatoid arthritis, colitis, pain from interstitial cystitis, visceral pain, pain from kidney stones, pain from gallstones, sore throat, fibromyalgia, chronic pain syndrome, thalamic pain syndrome, pain from stroke, phantom limb pain, sunburn, radiculopathy, complex regional pain syndrome, HIV sensory neuropathy, central pain syndrome, multiple sclerosis pain, Parkinson's disease pain, spinal cord injury pain, dysmenorrhea, toothache, pain from bone metastasis, pain from endometriosis, pain from uterine fibroids, nociceptive pain, hyperalgesia, temporomandibular joint pain, neuroblastoma, ovarian cancer, endometrial cancer, glioblastoma multiforme, cervical cancer, pancreatic cancer, colon cancer, rectal cancer, prostate cancer, melanoma, myeloma, thyroid cancer, lung cancer (small cell lung cancer, non-small cell lung cancer), brain tumor, esophageal cancer, kidney cancer, bone tumor, hematological cancer (chronic myelogenous leukemia, acute lymphoblastic leukemia, Philadelphia chromosome-positive acute lymphoblastic leukemia (Ph+ALL), acute myeloid leukemia (AML), chronic lymphocytic leukemia (CML)), squamous cell carcinoma, glioma, gastrointestinal cancer, ovarian cancer, liver cancer, stomach cancer, bladder cancer, hepatocellular carcinoma, breast cancer, head and neck cancer, germ cell tumor, pediatric sarcoma, extranodal NK/T-cell lymphoma, nasal type, multiple myeloma, interstitial cystitis (IC), painful bladder syndrome (PBS), urinary incontinence, inflammatory cystitis, inflammatory bowel disease, ulcerative colitis, Crohn's disease, systemic lupus erythematosus, rheumatoid arthritis, joint swelling, asthma, atopic dermatitis, psoriasis, psoriatic arthritis, rhinitis, systemic pruritus, localized pruritus, generalized pruritus, multiple sclerosis, Parkinson's disease, Alzheimer's disease, Chagas disease, Sjögren's syndrome, endometriosis, diabetic nephropathy, renal fibrosis, chronic kidney disease, osteoporosis, etc.

The oral solid formulation of the present invention can be used in subjects in need of prevention and/or treatment of diseases in which TrkA is involved. The subject is a human or non-human mammal (dog, cat, rat, mouse, monkey, cattle, horse, pig, sheep, etc.).

In the present invention, the content of 1-((1R,2R)-2-hydroxy-4,4-dimethyl-1,2,3,4-tetrahydronaphthalen-1-yl)-3-(2-phenylpyridin-3-yl)urea (compound (I)), or a pharmaceutically acceptable salt thereof, or a solvate thereof, contained in the oral solid formulation is not particularly limited as long as it is an amount that is effective in preventing or/and treating a disease in which TrkA is involved, but for example, when compound (I) is contained, 5 mg, 10 mg, 20 mg, 30 mg, 50 mg, 100 mg, 150 mg, etc., and amounts in a range having these as the upper limit or lower limit are mentioned. Furthermore, when a pharmaceutically acceptable salt of compound (I), a solvate of compound (I), or a solvate of a pharmaceutically acceptable salt of compound (I) is contained, each of these contents is, for example, an equivalent amount of the salt, solvate, or salt solvate corresponding to the amount of compound (I).

In the present invention, for example, when an oral solid formulation containing 50 mg or 150 mg of compound (I), or a pharmaceutically acceptable salt thereof in an amount equivalent to the amount, or a solvate thereof in an amount equivalent to the amount, is administered to an administration subject, the concentration of compound (I) in the plasma of the subject becomes about 90 nM or more, about 95 nM or more, about 100 nM or more, about 105 nM or more, or about 110 nM or more 12 hours or 24 hours after the initial administration.

The oral solid formulation of the present invention may be a formulation in which cracks in the formulation are prevented. Usually, tablets or cores are produced by compression-molding drug powder, but cracks (this phenomenon is sometimes called cracking, lamination, capping, etc.) may occur inside the tablets or cores during molding, depending on the production conditions, the type and content of additives to be included, and the like. For example, in the examples described later, the formulation of Example 1-2 containing 50 mg of compound (I) does not generate cracks in its production process, but when producing a formulation containing 150 mg of compound (I) using the formulation composition (Reference Example 1), cracks were confirmed in the core production stage. The cause of the cracks was considered to be insufficient binding force of the drug powder and poor release from the die; to solve these problems, in the formulation of Example 2-1, the excipient was changed to a mixture of mannitol and crystalline cellulose to increase the binding force of the drug powder, and the flow agent was changed to a mixture of light anhydrous silicic acid and talc to improve release, thereby enabling molding of tablets or cores having a hardness that does not show cracks even when the content of compound (I) in the formulation is up to 150 mg.

The method for producing the oral solid formulation of the present invention will be described below, but includes, for example, known methods including steps such as pulverization, mixing, granulation, drying, sieving, sizing, molding (tableting), coating, and crystallization.

The pulverization step is not particularly limited in terms of apparatus or means, as long as it is a method that can usually pharmaceutically pulverize a drug and appropriate additives. Examples of pulverizers include impact pulverizers, hammer mills, ball mills, jet mills, and colloid mills. The pulverization conditions are appropriately selected and are not particularly limited. The mixing step of each component subsequent to the pulverization is not particularly limited in terms of apparatus or means, as long as it is a method that can usually pharmaceutically mix each component uniformly.

Granulation may be used as a method for producing the oral solid formulation of the present invention. In the granulation step, the apparatus and means are not particularly limited as long as it is a method that can usually pharmaceutically granulate the drug and appropriate pharmaceutical additives. Examples of granulation methods and apparatuses used in wet granulation using water or a binder liquid in which an appropriate amount of binder is dispersed or dissolved in water or the like include, but are not limited to, high-speed stirring granulation methods, crushing (pulverizing) granulation methods, fluidized bed granulation methods, extrusion granulation methods, tumbling granulation methods, spray granulation methods, or apparatuses used by these methods. As a method that does not use water during granulation, a wet granulation method using a non-aqueous solvent or a dry granulation method that does not use a solvent can also be selected.

In the drying step, the apparatus and means are not particularly limited as long as it is a method that can usually be pharmaceutically dried. Examples of the apparatus include, but are not limited to, air-circulating dryers, reduced-pressure dryers, vacuum dryers, and fluidized-bed granulation dryers.

In the sieving and sizing steps, the apparatus and means are not particularly limited as long as it is a method that can usually be pharmaceutically sieved or sized. Examples of the apparatus include, but are not limited to, sieves, co-mills, and power mills.

In the compression molding step (tableting step), the apparatus and means are not particularly limited as long as it is a method for molding the formulation or core of the present invention. Examples include, but are not limited to, a method of producing a pharmaceutical composition by directly compression-molding a drug and appropriate pharmaceutical additives after mixing without performing a granulation/drying step, a method of producing a pharmaceutical composition by compression-molding after granulating and drying, and a method of producing a pharmaceutical composition (for example, uncoated tablets or cores) by compression-molding after granulating and further mixing a glidant.

Examples of tableting machines include rotary tableting machines, single-punch tableting machines, and oil presses.

The tableting conditions such as tableting pressure are not particularly limited as long as the core can be formed and the core is not damaged during the production process. For example, when compression molding is performed with a table-top rotary tableting machine (PICCOLA NOVA, manufactured by RIVA S.A.), the tableting pressure (main compression force) is 0.5 to 20.0 kN, preferably 1.0 to 10.0 kN, and more preferably 3.0 to 8.0 kN. The above upper and lower limits can be arbitrarily combined as desired.

Furthermore, the hardness of the core is not particularly limited as long as it is a hardness that does not break in the coating step. For example, when the hardness of a tablet is measured with a table-top rotary tableting machine (PICCOLA NOVA, manufactured by RIVA S.A.), the hardness is 30 N or more, preferably 40 N or more, and more preferably 50 N or more.

As a coating step, it is possible to appropriately coat the core surface after tableting.

The method is not particularly limited as long as it is a method that can usually be pharmaceutically coated. Examples include pan coating and dip coating.

The coating agent can be suitably added in an appropriate amount by combining one type or two or more types. The coating rate is not particularly limited as long as it is a rate that forms a film on the core. For example, it is 1% by mass to 20% by mass, preferably 2% by mass to 18% by mass, and more preferably 3% by mass to 16% by mass, based on the total mass of the tablet.

It may be dried after coating. The method is not particularly limited as long as it is a method that can usually be pharmaceutically dried.

### Examples

Hereinafter, the present invention will be described in more detail with reference to Examples, Comparative Examples, Reference Examples, and Test Examples, but the present invention is not limited thereto.

1-((1R,2R)-2-Hydroxy-4,4-dimethyl-1,2,3,4-tetrahydronaphthalen-1-yl)-3-(2-phenylpyridin-3-yl)urea described in the present Examples and the like was produced by the method described in WO 2018/199166 pamphlet (Patent Literature 1) or WO 2019/189717 pamphlet, or a method equivalent thereto. The contents described in WO 2018/199166 pamphlet and WO 2019/189717 pamphlet are incorporated herein as part of this specification.

The additives described in these Examples and the like were those listed in Table 2, respectively.

**Table 2**

| | |
|---|---|
| Additive | Product Name (Manufacturer Name) |
| Mannitol | Parteck M100 (manufactured by Merck) |
| | Parteck M200 (manufactured by Merck) |
| Crystalline cellulose | Ceolus KG-1000 (manufactured by Asahi Kasei Corporation) |
| | Ceolus UF-702 (manufactured by Asahi Kasei Corporation) |
| Granulated lactose | Dilactose (registered trademark) F (manufactured by Freund Corporation) |
| Anhydrous calcium hydrogen phosphate | Fujicalin (registered trademark) SG (manufactured by Fuji Chemical Industry Co., Ltd.) |
| Light anhydrous silicic acid | Adsolider 101 (manufactured by Freund Corporation) |
| Talc | HI-FILLER #17 (manufactured by Matsumura Sangyo Co., Ltd.) |
| Magnesium aluminometasilicate | Neusilin UFL2 (manufactured by Fuji Chemical Industry Co., Ltd.) |
| Croscarmellose sodium | Ac-Di-Sol (manufactured by FMC BioPolymer) |
| Sodium starch glycolate | EXPLOTAB (registered trademark) (manufactured by JRS Pharma) |
| Crospovidone | Polyplasdone XL (manufactured by Ashland) |
| Magnesium stearate | Pharmacopeial Magnesium Stearate (Vegetable) (manufactured by Taihei Chemical Industrial) |
| Sodium stearyl fumarate | Alubra (registered trademark) PG-100 (manufactured by DuPont) |
| Dry methacrylic acid copolymer LD | EUDRAGIT L100-55 (manufactured by Evonik Japan Co., Ltd.) |
| Methacrylic acid copolymer L | EUDRAGIT L100 (manufactured by Evonik Japan Co., Ltd.) |
| Methacrylic acid copolymer S | EUDRAGIT S100 (manufactured by Evonik Japan Co., Ltd.) |
| Aminoalkyl methacrylate copolymer E | EUDRAGIT E100 (manufactured by Evonik Japan Co., Ltd.) |
| Hypromellose phthalate | HPMCP (registered trademark) HP-50 (manufactured by Shin-Etsu Chemical Co., Ltd.) |
| Hypromellose acetate succinate | AQOAT (registered trademark) AS-MG (manufactured by Shin-Etsu Chemical Co., Ltd.): Succinyl group content 10-14%, pH solubility≥6.0 type |
| | AQOAT (registered trademark) AS-HG (manufactured by Shin-Etsu Chemical Co., Ltd.): Succinyl group content 4-8%, pH solubility≥6.5 type |
| Carboxymethyl ethyl cellulose | CMEC (manufactured by Freund Corporation) |
| Cellulose acetate phthalate | Cellacefate (manufactured by Eastman Chemical Japan) |
| Triethyl citrate | CITROFLEX (registered trademark) 2 (SC-60) (manufactured by Morimura Bros., Inc.) |
| Titanium dioxide | Titanium Dioxide NA65 (manufactured by Toho Titanium) |
| Anhydrous ethanol | Ethanol (99.5) (manufactured by FUJIFILM Wako Pure Chemical Corporation) |

### <Production of Formulation (Tablet) of Comparative Example 1>

According to the formulation shown in Table 3 below, 4.00 g of light anhydrous silicic acid was added to 22.18 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 163.82 g of mannitol and 8.00 g of croscarmellose sodium were added to the obtained mixture, manually mixed using a polyethylene bag, sieved to break up agglomerates, and further 2.00 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a formulation (tablet) of Comparative Example 1.

### <Production of Formulation (Tablet) of Example 1-1>

According to the formulation shown in Table 3 below, a solid formulation was prepared using 220 g of the formulation of Comparative Example 1 as a core. Specifically, the core was placed in a film coating machine (PRC-GTX-mini, manufactured by Powrex Corporation), and film-coated with a liquid in which EUDRAGIT L100-55, triethyl citrate, and talc were dispersed or dissolved in anhydrous ethanol and purified water to obtain the formulation (tablet) of Example 1-1.

### <Production of Formulation (Tablet) of Example 1-2>

According to the formulation shown in Table 3 below, 22.0 g of light anhydrous silicic acid was added to 260.6 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 762.5 g of mannitol and 44.00 g of croscarmellose sodium were added to the obtained mixture, manually mixed using a polyethylene bag, sieved to break up agglomerates, and further 2.00 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 219.8 g of the obtained core was placed in a film coating machine (PRC-GTX-mini, manufactured by Powrex Corporation), and film-coated with a liquid in which EUDRAGIT L100-55, triethyl citrate, and talc were dispersed or dissolved in anhydrous ethanol and purified water to obtain the formulation (tablet) of Example 1-2.

### <Production of Formulation of Reference Example 1>

According to the formulation shown in Table 3 below, the formulation of Reference Example 1 is obtained according to the method of Example 1-2.

**Table 3**

| Ingredient | Comparative Example 1 | Example 1-1 | Example 1-2 | Reference Example 1 |
|---|---|---|---|---|
| Compound (I) | 23.4 | 23.4 | 50.0 | 150.0 |
| Mannitol (Parteck M100) | - | - | 154.6 | 463.8 |
| Mannitol (Parteck M200) | 181.2 | 181.2 | - | - |
| Light anhydrous silicic acid | 4.4 | 4.4 | 4.4 | 13.2 |
| Croscarmellose sodium (Ac-Di-Sol) | 8.8 | 8.8 | 8.8 | 26.4 |
| Magnesium stearate | 2.2 | 2.2 | 2.2 | 6.6 |
| Core portion subtotal | 220 | 220 | 220 | 660 |
| Dry methacrylic acid copolymer LD (EUDRAGIT L100-55) | - | 9.43 | 9.43 | 16.4 |
| Triethyl citrate | - | 1.89 | 1.89 | 3.3 |
| Talc | - | 1.89 | 1.89 | 3.3 |
| Anhydrous ethanol | - | 0 | 0 | 0 |
| Purified water | - | 0 | 0 | 0 |
| Coating portion subtotal | - | 13.21 | 13.21 | 23 |
| Total | 220 | 233.21 | 233.21 | 683 |

| | | | | |
|---|---|---|---|---|
| (The unit of numerical values is mg) | | | | |

### <Test Example 1> Dissolution Test (1)

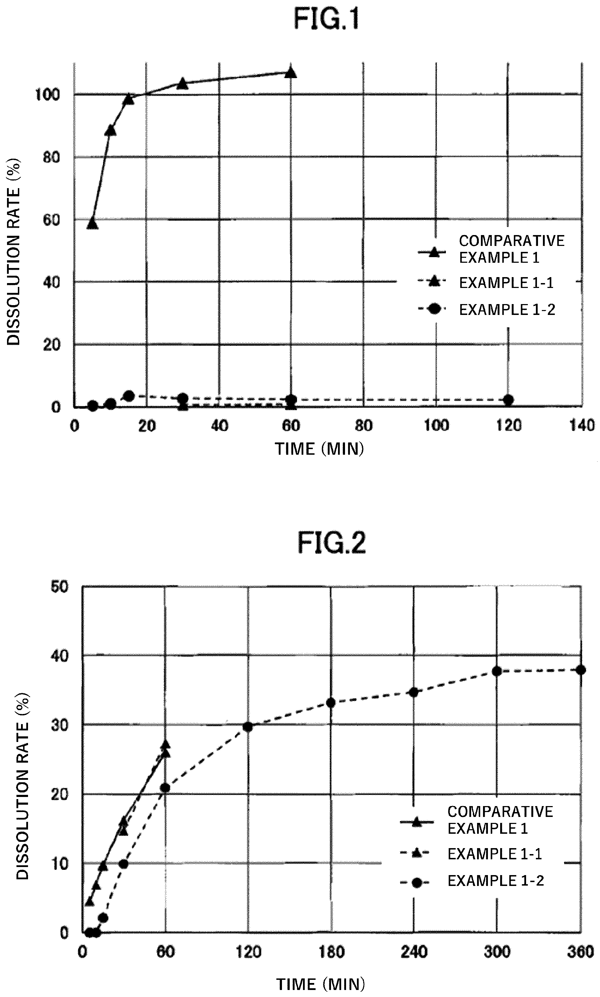

For each formulation produced in Comparative Example 1, Example 1-1, and Example 1-2, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 1 (JP1 solution) as the dissolution test solution, and a paddle rotation speed of 50 revolutions/minute. For Comparative Example 1, 5, 10, 15, 30, and 60 minutes after the start of the test, for Example 1-1, 30 and 60 minutes after the start of the test, and for Example 1-2, 5, 10, 15, 30, 60, and 120 minutes after the start of the test, the absorbance of 1-((1R,2R)-2-hydroxy-4,4-dimethyl-1,2,3,4-tetrahydronaphthalen-1-yl)-3-(2-phenylpyridin-3-yl)urea in the dissolution test solution was measured using ultraviolet-visible spectrophotometry (UV method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained absorbance (the start of the test is set to a dissolution rate of 0%. The same applies to the following dissolution rate results). As to the UV method, measurement was performed using an ultraviolet-visible spectrophotometer (V-660, manufactured by JASCO Corporation) (wavelength: 210 nm). The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 4 and Fig. 1.

**Table 4**

| Formulation (tablets) | Sampling time (min) | | | | | |
|---|---|---|---|---|---|---|
| | 5 min | 10 min | 15 min | 30 min | 60 min | 120 min |
| Comparative Example 1 (%) | 58.9 | 88.8 | 98.7 | 103.6 | 107 | - |
| Example 1-1 (%) | - | - | - | 0.6 | 0.7 | - |
| Example 1-2 (%) | 0.4 | 1 | 3.5 | 2.7 | 2.3 | 2.1 |

### <Test Example 2> Dissolution Test (2)

For each formulation produced in Comparative Example 1, Example 1-1, and Example 1-2, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 2 (JP2 solution) as the dissolution test solution, and a paddle rotation speed of 50 revolutions/minute. For Comparative Example 1 and Example 1-1, 5, 10, 15, 30, 60, and 120 minutes after the start of the test, and for Example 1-2, 5, 10, 15, 30, 60, 120, 180, 240, 300, and 360 minutes after the start of the test, the absorbance of 1-((1R,2R)-2-hydroxy-4,4-dimethyl-1,2,3,4-tetrahydronaphthalen-1-yl)-3-(2-phenylpyridin-3-yl)urea in the dissolution test solution was measured using ultraviolet-visible spectrophotometry (UV method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained absorbance. As to the UV method, measurement was performed using an ultraviolet-visible spectrophotometer (V-660, manufactured by JASCO Corporation) (wavelength: 210 nm). The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 5 and Fig. 2.

**Table 5**

| Formulation (tablets) | Sampling time (min) | | | | | | | | | |
|---|---|---|---|---|---|---|---|---|---|---|
| | 5 min | 10 min | 15 min | 30 min | 60 min | 120 min | 180 min | 240 min | 300 min | 360 min |
| Comparative Example 1 (%) | 4.5 | 6.9 | 9.7 | 16.2 | 26 | 42 | - | - | - | - |
| Example 1-1 (%) | 0.5 | 1.2 | 4.7 | 14.7 | 27.3 | 46.5 | - | - | - | - |
| Example 1-2 (%) | 0 | 0 | 2.1 | 9.9 | 20.9 | 29.7 | 33.2 | 34.7 | 37.7 | 37.9 |

### <Production of Formulation (Tablet) of Example 2-1>

According to the formulation shown in Table 6 below, 22.0 g of light anhydrous silicic acid was added to 255.00 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 707.51 g of mannitol, 5.50 g of talc, 44.00 g of croscarmellose sodium, and 55.00 g of crystalline cellulose were added to the obtained mixture, manually mixed using a polyethylene bag, sieved to break up agglomerates, and further 11.00 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 500.10 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 2-1.

### <Production of Formulation (Tablet) of Example 2-2>

According to the formulation shown in Table 6 below, 66.00 g of light anhydrous silicic acid was added to 767.50 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 2120.00 g of mannitol, 16.50 g of talc, 132.00 g of croscarmellose sodium, and 165.00 g of crystalline cellulose were added to the obtained mixture, manually mixed using a polyethylene bag, sieved to break up agglomerates, and further 33.00 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 600.56 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 2-2.

### <Production of Cores of Examples 2-3, 2-4, and 2-5>

According to the formulation shown in Table 6 below, 66.0 g of light anhydrous silicic acid was added to 779.8 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 2107.7 g of mannitol, 16.5 g of talc, 132.0 g of croscarmellose sodium, and 165.0 g of crystalline cellulose were added to the obtained mixture, manually mixed using a polyethylene bag, sieved to break up agglomerates, and further 33.0 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core.

### <Production of Formulations (Tablets) of Examples 2-3, 2-4, and 2-5>

600 g of the core obtained in <Production of Core Tablets of Examples 2-3, 2-4, and 2-5> above was placed in a film coating machine (PRC-GTX-mini, manufactured by Powrex Corporation), and for Example 2-3, film-coated with a liquid in which hypromellose phthalate, triethyl citrate, talc, and titanium dioxide were dispersed or dissolved in anhydrous ethanol and purified water, and for Examples 2-4 and 2-5, film-coated with a liquid in which hypromellose acetate succinate, triethyl citrate, talc, and titanium dioxide were dispersed or dissolved in anhydrous ethanol and purified water to obtain formulations (tablets) of Examples 2-3, 2-4, and 2-5.

**Table 6**

| Ingredient | Example 2-1 | Example 2-2 | Example 2-3 | Example 2-4 | Example 2-5 |
|---|---|---|---|---|---|
| Compound (I) | 50.0 | 150.0 | 50.0 | 50.0 | 50.0 |
| Mannitol (Parteck M100) | 142.5 | 427.5 | 142.5 | 142.5 | 142.5 |
| Crystalline cellulose (Ceolus KG-1000) | 11 | 33 | 11 | 11 | 11 |
| Light anhydrous silicic acid | 4.4 | 13.2 | 4.4 | 4.4 | 4.4 |
| Talc | 1.1 | 3.3 | 1.1 | 1.1 | 1.1 |
| Croscarmellose sodium (Ac-Di-Sol) | 8.8 | 26.4 | 8.8 | 8.8 | 8.8 |
| Magnesium stearate | 2.2 | 6.6 | 2.2 | 2.2 | 2.2 |
| Core portion subtotal | 220 | 660 | 220 | 220 | 220 |
| Dry methacrylic acid copolymer LD (EUDRAGIT L100-55) | 7.9 | 16.4 | - | - | - |
| Hypromellose phthalate (HPMCP) | - | - | 7.9 | - | - |
| Hypromellose acetate succinate (AQOAT-MG) | - | - | - | 7.9 | - |
| Hypromellose acetate succinate (AQOAT-HG) | - | - | - | - | 7.9 |
| Triethyl citrate | 1.6 | 3.3 | 1.6 | 1.6 | 1.6 |
| Talc | 1.6 | 3.3 | 1.6 | 1.6 | 1.6 |
| Titanium dioxide | 1.9 | 4 | 1.9 | 1.9 | 1.9 |
| Anhydrous ethanol | 0 | 0 | 0 | 0 | 0 |
| Purified water | 0 | 0 | 0 | 0 | 0 |
| Coating portion subtotal | 13 | 27 | 13 | 13 | 13 |
| Total | 233 | 687 | 233 | 233 | 233 |

| | | | | | |
|---|---|---|---|---|---|
| (The unit of numerical values is mg) | | | | | |

### <Test Example 3> Dissolution Test (3)

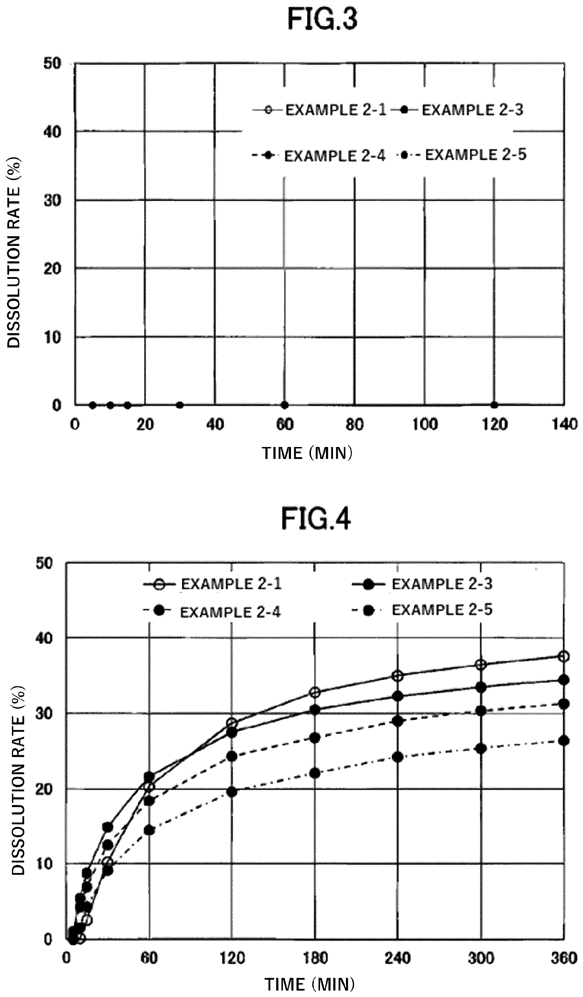

For each formulation (tablet) produced in Examples 2-1, 2-3, 2-4, and 2-5, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 1 (JP1 solution) as the dissolution test solution, and a paddle rotation speed of 50 revolutions/minute. 5, 10, 15, 30, 60, and 120 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 7 and Fig. 3.

**Table 7**

| Formulation (tablets) | Sampling time (min) | | | | | |
|---|---|---|---|---|---|---|
| | 5 min | 10 min | 15 min | 30 min | 60 min | 120 min |
| Example 2-1 (%) | 0 | 0 | 0 | 0 | 0.1 | 0 |
| Example 2-3 (%) | 0 | 0 | 0 | 0 | 0 | 0 |
| Example 2-4 (%) | 0 | 0 | 0 | 0 | 0 | 0 |
| Example 2-5 (%) | 0 | 0 | 0 | 0 | 0 | 0 |

### <Test Example 4> Dissolution Test (4)

For each formulation (tablet) produced in Examples 2-1, 2-3, 2-4, and 2-5, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 2 (JP2 solution) as the dissolution test solution, and a paddle rotation speed of 50 revolutions/minute. 5, 10, 15, 30, 60, 120, 180, 240, 300, and 360 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 8 and Fig. 4.

**Table 8**

| Formulation (tablets) | Sampling time (min) | | | | | | | | | |
|---|---|---|---|---|---|---|---|---|---|---|
| | 5 min | 10 min | 15 min | 30 min | 60 min | 120 min | 180 min | 240 min | 300 min | 360 min |
| Example 2-1 (%) | 0 | 0.1 | 2.5 | 10.2 | 20.3 | 28.7 | 32.8 | 35 | 36.5 | 37.6 |
| Example 2-3 (%) | 1 | 5.4 | 8.7 | 14.9 | 21.6 | 27.5 | 30.5 | 32.3 | 33.5 | 34.5 |
| Example 2-4 (%) | 0.5 | 4.2 | 6.9 | 12.5 | 18.4 | 24.3 | 26.8 | 29 | 30.4 | 31.3 |
| Example 2-5 (%) | 0 | 1.5 | 4.2 | 9.1 | 14.5 | 19.6 | 22.1 | 24.2 | 25.4 | 26.4 |

### <Production of Formulations (Tablets) of Examples 3-1 and 3-2>

According to the formulation shown in Table 9 below, 17.6 g of light anhydrous silicic acid was added to 207.9 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 562.1 g of mannitol, 4.4 g of talc, 35.2 g of croscarmellose sodium, and 44.0 g of crystalline cellulose were added to the obtained mixture, manually mixed using a polyethylene bag, sieved to break up agglomerates, and further 8.8 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 600.1 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain formulations (tablets) of Examples 3-1 and 3-2.

**Table 9**

| Ingredient | Example 3-1 | Example 3-2 |
|---|---|---|
| Compound (I) | 50.0 | 50.0 |
| Mannitol (Parteck M100) | 142.5 | 142.5 |
| Crystalline cellulose (Ceolus KG-1000) | 11 | 11 |
| Light anhydrous silicic acid | 4.4 | 4.4 |
| Talc | 1.1 | 1.1 |
| Croscarmellose sodium (Ac-Di-Sol) | 8.8 | 8.8 |
| Magnesium stearate | 2.2 | 2.2 |
| Core portion subtotal | 220 | 220 |
| Dry methacrylic acid copolymer LD (EUDRAGIT L100-55) | 15.8 | 23.7 |
| Triethyl citrate | 3.2 | 4.8 |
| Talc | 3.2 | 4.8 |
| Titanium dioxide | 3.8 | 5.7 |
| Anhydrous ethanol | 0 | 0 |
| Purified water | 0 | 0 |
| Coating portion subtotal | 26 | 39 |
| Total | 246 | 259 |

| | | |
|---|---|---|
| (The unit of numerical values is mg) | | |

### <Test Example 5> Dissolution Test (5)

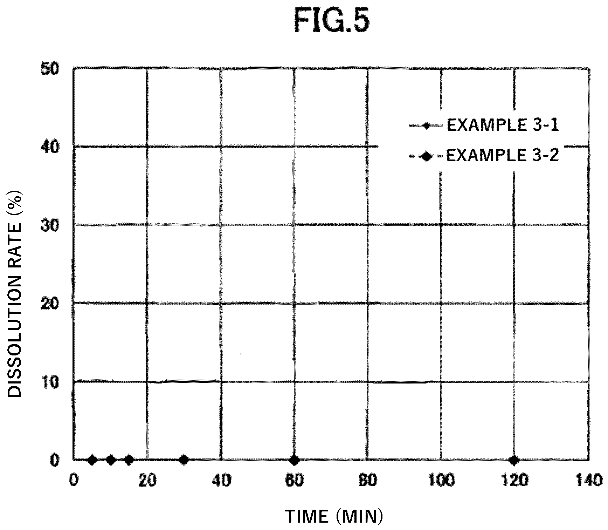

For each formulation (tablet) produced in Examples 3-1 and 3-2, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 1 (JP1 solution) as the dissolution test solution, and a paddle rotation speed of 50 revolutions/minute. 5, 10, 15, 30, 60, and 120 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 10 and Fig. 5.

**Table 10**

| Formulation (tablets) | Sampling time (min) | | | | | |
|---|---|---|---|---|---|---|
| | 5 min | 10 min | 15 min | 30 min | 60 min | 120 min |
| Example 3-1 (%) | 0 | 0 | 0 | 0 | 0 | 0 |
| Example 3-2 (%) | 0 | 0 | 0 | 0 | 0 | 0 |

### <Test Example 6> Dissolution Test (6)

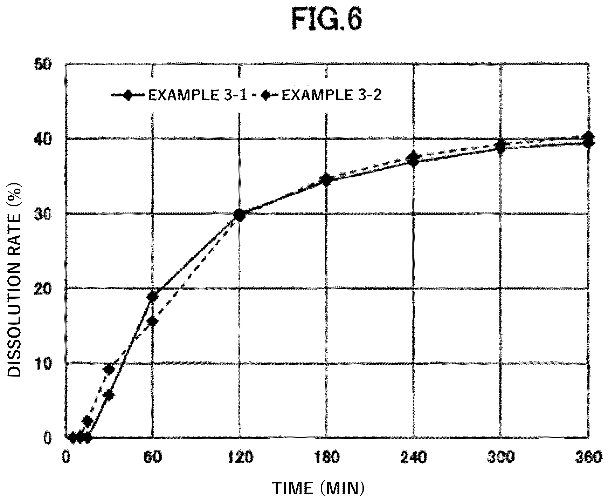

For each formulation (tablet) produced in Examples 3-1 and 3-2, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 2 (JP2 solution) as the dissolution test solution, and a paddle rotation speed of 50 revolutions/minute. 5, 10, 15, 30, 60, 120, 180, 240, 300, and 360 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 11 and Fig. 6.

**Table 11**

| Formulation (tablets) | Sampling time (min) | | | | | | | | | |
|---|---|---|---|---|---|---|---|---|---|---|
| | 5 min | 10 min | 15 min | 30 min | 60 min | 120 min | 180 min | 240 min | 300 min | 360 min |
| Example 3-1 | 0 | 0 | 0 | 5.7 | 18.9 | 30 | 34.3 | 36.9 | 38.7 | 39.5 |
| (%) | | | | | | | | | | |
| Example 3-2 (%) | 0 | 0.2 | 2.2 | 9.2 | 15.6 | 29.7 | 34.7 | 37.6 | 39.3 | 40.3 |

### <Production of Formulation (Tablet) of Example 4-1>

According to the formulation shown in Table 12 below, 4.4 g of light anhydrous silicic acid was added to 51.1 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 141.4 g of lactose monohydrate, 1.10 g of talc, 8.8 g of croscarmellose sodium, and 11.0 g of crystalline cellulose were added to the obtained mixture, manually mixed using a polyethylene bag, sieved to break up agglomerates, and further 2.2 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 130.0 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 4-1.

### <Production of Formulation (Tablet) of Example 4-2>

According to the formulation shown in Table 12 below, 4.4 g of light anhydrous silicic acid was added to 51.1 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 141.4 g of crystalline cellulose, 1.10 g of talc, 8.8 g of croscarmellose sodium, and 11.0 g of crystalline cellulose were added to the obtained mixture, manually mixed using a polyethylene bag, sieved to break up agglomerates, and further 2.2 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 150.1 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 4-2.

### <Production of Formulation (Tablet) of Example 4-3>

According to the formulation shown in Table 12 below, 4.4 g of magnesium aluminometasilicate was added to 51.1 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 141.4 g of mannitol, 1.10 g of talc, 8.8 g of croscarmellose sodium, and 11.0 g of crystalline cellulose were added to the obtained mixture, manually mixed using a polyethylene bag, sieved to break up agglomerates, and further 2.2 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 140.1 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 4-3.

### <Production of Formulation (Tablet) of Example 4-4>

According to the formulation shown in Table 12 below, 4.4 g of light anhydrous silicic acid was added to 51.1 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 141.4 g of mannitol, 1.10 g of talc, 8.8 g of sodium starch glycolate, and 11.0 g of crystalline cellulose were added to the obtained mixture, manually mixed using a polyethylene bag, sieved to break up agglomerates, and further 2.2 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core. 150.0 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 4-4.

### <Production of Formulation (Tablet) of Example 4-5>

According to the formulation shown in Table 12 below, 4.4 g of light anhydrous silicic acid was added to 51.1 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 139.2 g of mannitol, 1.10 g of talc, 8.8 g of croscarmellose sodium, and 11.0 g of crystalline cellulose were added to the obtained mixture, manually mixed using a polyethylene bag, sieved to break up agglomerates, and further 4.4 g of sodium stearyl fumarate was added and manually mixed to obtain a mixed powder for tableting. The obtained mixed powder for tableting was tableted using a table-top rotary tableting machine (PICCOLA NOVA, manufactured by RIVA S.A.) to obtain a core. 150.1 g of the obtained core was placed in a film coating machine (PRC-GTX-mini, manufactured by Powrex Corporation), and film-coated with a liquid in which EUDRAGIT L100-55, triethyl citrate, talc, and titanium dioxide were dispersed or dissolved in anhydrous ethanol and purified water to obtain the formulation (tablet) of Example 4-5.

**Table 12**

| Ingredient | Example 4-1 | Example 4-2 | Example 4-3 | Example 4-4 | Example 4-5 |
|---|---|---|---|---|---|
| Compound (I) | 50.0 | 50.0 | 50.0 | 50.0 | 50.0 |
| Mannitol (Parteck M100) | - | - | 142.5 | 142.5 | 140.3 |
| Crystalline cellulose (Ceolus KG-1000) | 11 | 11 | 11 | 11 | 11 |
| Granulated lactose (Dilactose F) | 142.5 | - | - | - | - |
| Crystalline cellulose (Ceolus UF-702) | - | 142.5 | - | - | - |
| Light anhydrous silicic acid | 4.4 | 4.4 | - | 4.4 | 4.4 |
| Talc | 1.1 | 1.1 | 1.1 | 1.1 | 1.1 |
| Magnesium aluminometasilicate (Neusilin UFL2) | - | - | 4.4 | - | - |
| Croscarmellose sodium (Ac-Di-Sol) | 8.8 | 8.8 | 8.8 | - | 8.8 |
| Sodium starch glycolate (Primojel) | - | - | - | 8.8 | - |
| Magnesium stearate | 2.2 | 2.2 | 2.2 | 2.2 | - |
| Sodium stearyl fumarate (PRUV) | - | - | - | - | 4.4 |
| Core portion subtotal | 220 | 220 | 220 | 220 | 220 |
| Dry methacrylic acid copolymer LD (EUDRAGIT L100-55) | 7.9 | 7.9 | 7.9 | 7.9 | 7.9 |
| Triethyl citrate | 1.6 | 1.6 | 1.6 | 1.6 | 1.6 |
| Talc | 1.6 | 1.6 | 1.6 | 1.6 | 1.6 |
| Titanium dioxide | 1.9 | 1.9 | 1.9 | 1.9 | 1.9 |
| Anhydrous ethanol | 0 | 0 | 0 | 0 | 0 |
| Purified water | 0 | 0 | 0 | 0 | 0 |
| Coating portion subtotal | 13 | 13 | 13 | 13 | 13 |
| Total | 233 | 233 | 233 | 233 | 233 |

| | | | | | |
|---|---|---|---|---|---|
| (The unit of numerical values is mg) | | | | | |

### <Test Example 7> Dissolution Test (7)

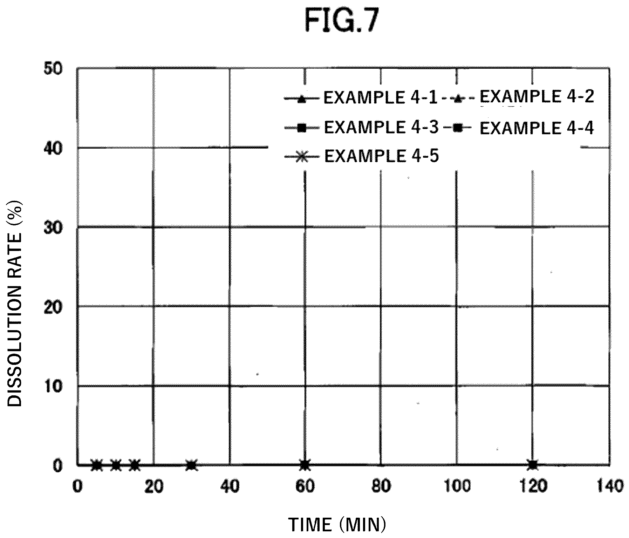

For each formulation (tablet) produced in Examples 4-1, 4-2, 4-3, 4-4, and 4-5, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 1 (JP1 solution) as the dissolution test solution, and a paddle rotation speed of 50 revolutions/minute. 5, 10, 15, 30, 60, and 120 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 13 and Fig. 7.

**Table 13**

| Formulation (tablets) | Sampling time (min) | | | | | |
|---|---|---|---|---|---|---|
| | 5 min | 10 min | 15 min | 30 min | 60 min | 120 min |
| Example 4-1 (%) | 0 | 0 | 0 | 0 | 0 | 0 |
| Example 4-2 (%) | 0 | 0 | 0 | 0 | 0 | 0 |
| Example 4-3 (%) | 0 | 0 | 0 | 0 | 0 | 0 |
| Example 4-4 (%) | 0 | 0 | 0 | 0 | 0 | 0 |
| Example 4-5 (%) | 0 | 0 | 0 | 0 | 0 | 0 |

### <Test Example 8> Dissolution Test (8)

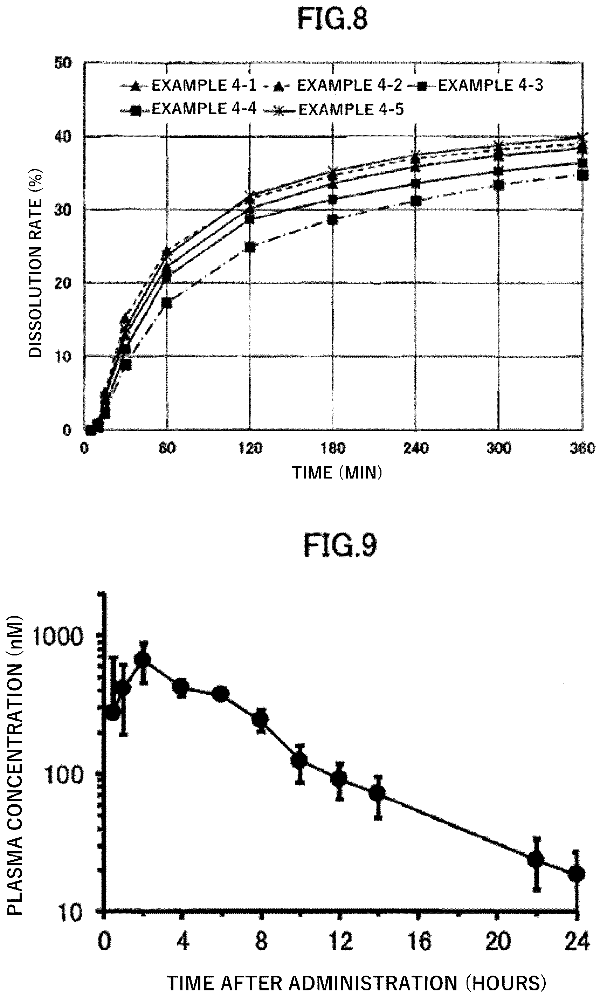

For each formulation (tablet) produced in Examples 4-1, 4-2, 4-3, 4-4, and 4-5, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 2 (JP2 solution) as the dissolution test solution, and a paddle rotation speed of 50 revolutions/minute. 5, 10, 15, 30, 60, 120, 180, 240, 300, and 360 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 14 and Fig. 8.

**Table 14**

| Formulation (tablets) | Sampling time (min) | | | | | | | | | |
|---|---|---|---|---|---|---|---|---|---|---|
| | 5 min | 10 min | 15 min | 30 min | 60 min | 120 min | 180 min | 240 min | 300 min | 360 min |
| Example 4-1 (%) | 0 | 1.1 | 4.1 | 12.9 | 22.2 | 30.2 | 33.6 | 35.9 | 37.4 | 38.4 |
| Example 4-2 (%) | 0 | 0.4 | 5.1 | 15.3 | 24.4 | 31.5 | 34.7 | 37 | 38.2 | 39 |
| Example 4-3 (%) | 0 | 0.8 | 2.6 | 11 | 20.8 | 28.7 | 31.4 | 33.6 | 35.3 | 36.4 |
| Example 4-4 (%) | 0 | 0.6 | 2.2 | 8.9 | 17.3 | 24.9 | 28.7 | 31.2 | 33.4 | 34.8 |
| Example 4-5 (%) | 0 | 0.6 | 4.5 | 13.8 | 23.7 | 31.9 | 35.3 | 37.5 | 38.8 | 39.9 |

Pharmacokinetics (PK) test:
Hereinafter, pharmacokinetics (PK) tests using the tablets (oral solid formulations) produced in Examples 1-1, 1-2, 2-1, 2-2, 2-3, 2-4, and 2-5, and Comparative Example 1 will be described.
In the graphs of Figs. 9 to 16, the vertical axis represents the plasma concentration (blood concentration) (nM) of compound (I), and the horizontal axis represents the time (hours) after administration of compound (I).

### (Comparative Example PK1)

### (1) Administration and blood collection of test formulation

An oral solid formulation (containing 23.4 mg of compound (I)) produced by the formulation prescription of Comparative Example 1 was orally administered to beagle dogs (male, 2 to 5 years old, 11.00 to 13.25 kg) under non-fasting conditions at a dose of 23.4 mg/body with water (30 mL) (n=3: 11.80 kg (5 years old), 11.00 kg (2 years old), 13.25 kg (2 years old)). Blood (0.5 mL) was collected from the cephalic vein of the beagle dogs at 0.5, 1, 2, 4, 6, 8, 10, 12, 14, 22, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

To plasma (0.02 mL) collected in (1) above, an internal standard solution (0.18 mL) of Buspirone (manufactured by Sigma-Aldrich) dissolved in methanol at 50 nmol/L was added and thoroughly stirred, and then allowed to stand on ice for 30 minutes. The mixed solution was centrifuged at 540 G for 5 minutes (Hitachi multi-tube refrigerated centrifuge CF-9RX, manufactured by Hitachi Koki Co., Ltd.), the supernatant was collected, and the concentration of compound (I) in plasma was measured using LC-MS/MS (Triple Quad 4500, manufactured by AB Sciex). The plasma concentration of compound (I) was calculated from a calibration curve prepared using standard plasma samples prepared by serially diluting a 1 mg/mL solution of compound (I) with plasma, and the plasma concentration transition data shown in Fig. 9 was obtained.

From Fig. 9, when the oral solid formulation produced by the formulation prescription of (Comparative Example PK1) was orally administered, the maximum plasma concentration (662 nM) was reached 2 hours after administration due to good absorption, and then rapidly decreased from plasma, and most of compound (I) in plasma disappeared 24 hours after administration (plasma concentration 12 hours after administration: 91.6 nM, plasma concentration 24 hours after administration: 18.6 nM). Furthermore, the maximum plasma concentration of this formulation is considered to increase dose-dependently, and it was predicted that the maximum plasma concentration would be about 1410 nM at a dose of 50 mg/body and about 4240 nM at a dose of 150 mg/body.

### (Example PK1-1)

### (1) Administration and blood collection of test formulation

An oral solid formulation (containing 23.4 mg of compound (I)) produced by the formulation prescription of Example 1-1 was orally administered to beagle dogs (male, 2 to 5 years old, 10.95 to 12.50 kg) under non-fasting conditions at a dose of 23.4 mg/body with water (30 mL) (n=3: 12.50 kg (5 years old), 12.00 kg (2 years old), 10.95 kg (2 years old)). Blood (0.5 mL) was collected from the cephalic vein of the beagle dogs at 0.5, 1, 2, 4, 6, 8, 10, 12, 14, 22, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

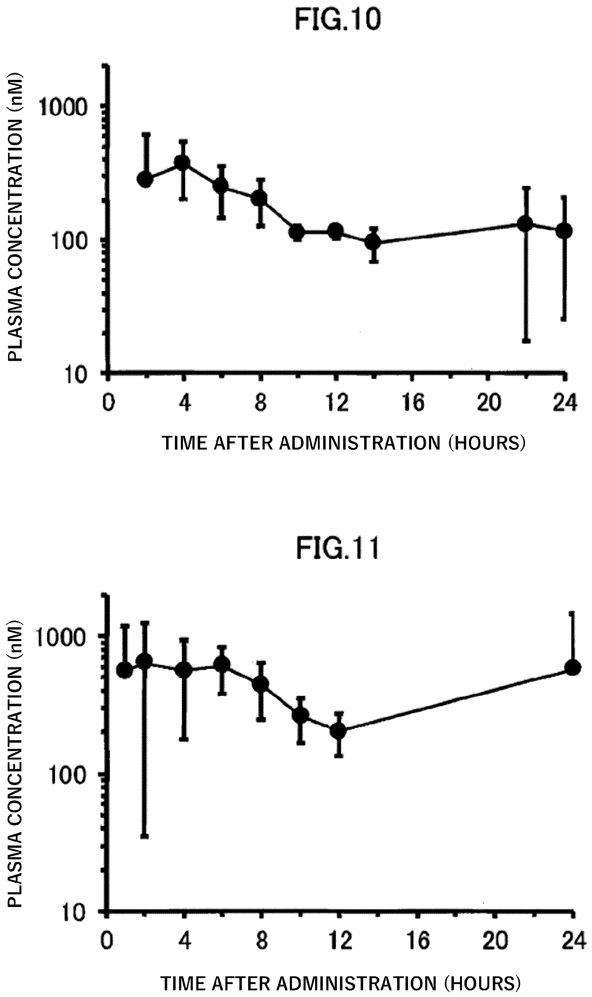

The same operation as the concentration measurement in (2) of Comparative Example PK1 was performed to calculate the plasma concentration of compound (I), and the plasma concentration transition data shown in Fig. 10 was obtained.

From Fig. 10, when the oral solid formulation produced by the formulation prescription of (Example 1-1) was orally administered, the maximum plasma concentration (374 nM) was reached 4 hours after administration, and the plasma concentration of compound (I) maintained a high concentration (plasma concentration 12 hours after administration: 114 nM, plasma concentration 24 hours after administration: 116 nM) even 12 hours after administration and beyond.

### (Example PK1-2)

### (1) Administration and blood collection of test formulation

An oral solid formulation (containing 50 mg of compound (I)) produced by the formulation prescription of Example 1-2 was orally administered to beagle dogs (male, 2 to 5 years old, 10.1 to 12.8 kg) under non-fasting conditions at a dose of 50 mg/body with water (30 mL) (n=6: 12.3 kg (5 years old), 10.1 kg (2 years old), 12.8 kg (2 years old), 12.8 kg (5 years old), 12.2 kg (2 years old), 10.6 kg (2 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

The same operation as the concentration measurement in (2) of Comparative Example PK1 was performed to calculate the plasma concentration of compound (I), and the plasma concentration transition data shown in Fig. 11 was obtained.

From Fig. 11, when the oral solid formulation produced by the formulation prescription of (Example 1-2) was orally administered, the maximum plasma concentration (634 nM) was reached 2 hours after administration, and the plasma concentration of compound (I) maintained a high concentration (plasma concentration 12 hours after administration: 202 nM, plasma concentration 24 hours after administration: 581 nM) even 12 hours after administration and beyond.

### (Example PK2-1)

### (1) Administration and blood collection of test formulation

An oral solid formulation (containing 50 mg of compound (I)) produced by the formulation prescription of Example 2-1 was orally administered to beagle dogs (male, 4 years old, 12.3 to 13.8 kg) under non-fasting conditions at a dose of 50 mg/body with water (30 mL) (n=4: 12.8 kg (4 years old), 13.8 kg (4 years old), 13.3 kg (4 years old), 12.3 kg (4 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

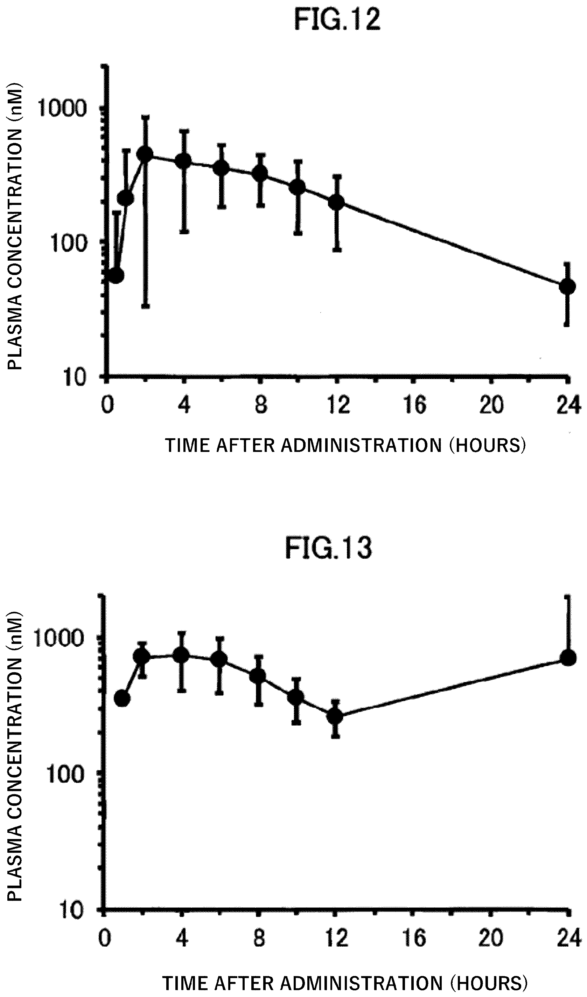

The same operation as the concentration measurement in (2) of Comparative Example PK1 was performed to calculate the plasma concentration of compound (I), and the plasma concentration transition data shown in Fig. 12 was obtained.

From Fig. 12, when the oral solid formulation produced by the formulation prescription of (Example 2-1) was orally administered, the maximum plasma concentration (440 nM) was reached 2 hours after administration, and the plasma concentration of compound (I) maintained a high concentration (plasma concentration 12 hours after administration: 195 nM, plasma concentration 24 hours after administration: 46.3 nM) even 12 hours after administration and beyond.

### (Example PK2-2)

### (1) Administration and blood collection of test formulation

An oral solid formulation (containing 150 mg of compound (I)) produced by the formulation prescription of Example 2-2 was orally administered to beagle dogs (male, 4 years old, 12.4 to 14.0 kg) under non-fasting conditions at a dose of 150 mg/body with water (30 mL) (n=4: 12.5 kg (4 years old), 14.0 kg (4 years old), 13.4 kg (4 years old), 12.4 kg (4 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

The same operation as the concentration measurement in (2) of Comparative Example PK1 was performed to calculate the plasma concentration of compound (I), and the plasma concentration transition data shown in Fig. 13 was obtained.

From Fig. 13, when the oral solid formulation produced by the formulation prescription of (Example 2-2) was orally administered, the maximum plasma concentration (735 nM) was reached 4 hours after administration, and the plasma concentration of compound (I) maintained a high concentration (plasma concentration 12 hours after administration: 258 nM, plasma concentration 24 hours after administration: 689 nM) even 12 hours after administration and beyond. Although the content of compound (I) in this formulation is 6.1 times higher than that of the oral solid formulation produced by the formulation prescription of Comparative Example 1, the maximum plasma concentration after administration is almost the same at 1.1 times, indicating suppression of a rapid increase in plasma concentration in the oral solid formulation produced by the formulation prescription of Example 2-2.

### (Example PK2-3)

### (1) Administration and blood collection of test formulation

An oral solid formulation (containing 50 mg of compound (I)) produced by the formulation prescription of Example 2-3 was orally administered to beagle dogs (male, 4 years old, 12.3 to 13.4 kg) under non-fasting conditions at a dose of 50 mg/body with water (30 mL) (n=4: 12.3 kg (4 years old), 13.4 kg (4 years old), 12.6 kg (4 years old), 12.8 kg (4 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

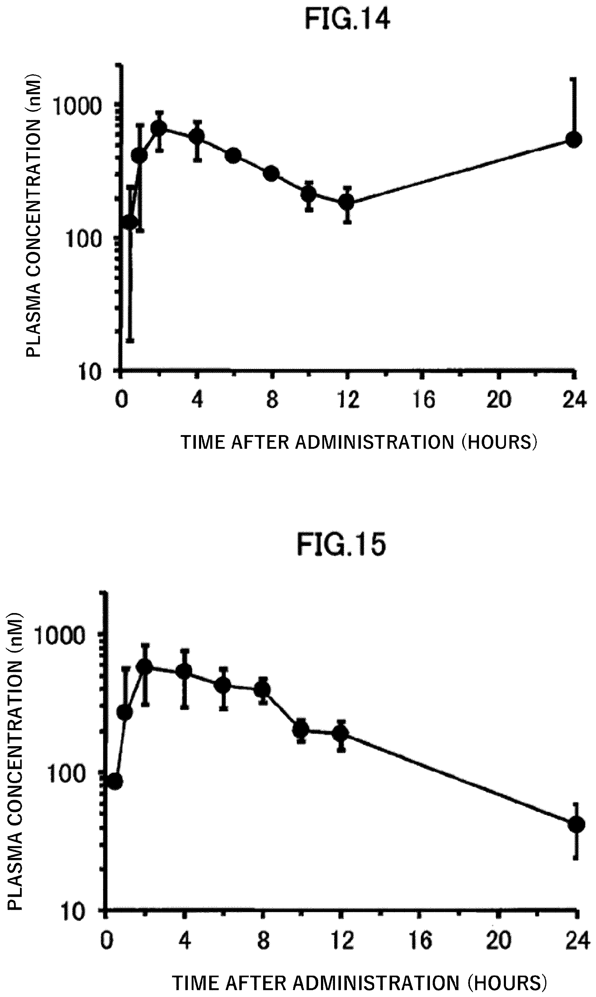

The same operation as the concentration measurement in (2) of Comparative Example PK1 was performed to calculate the plasma concentration of compound (I), and the plasma concentration transition data shown in Fig. 14 was obtained.

From Fig. 14, when the oral solid formulation produced by the formulation prescription of (Example 2-3) was orally administered, the maximum plasma concentration (664 nM) was reached 2 hours after administration, and the plasma concentration of compound (I) maintained a high concentration (plasma concentration 12 hours after administration: 184 nM, plasma concentration 24 hours after administration: 552 nM) even 12 hours after administration and beyond.

### (Example PK2-4)

### (1) Administration and blood collection of test formulation

An oral solid formulation (containing 50 mg of compound (I)) produced by the formulation prescription of Example 2-4 was orally administered to beagle dogs (male, 5 years old, 12.15 to 13.00 kg) under non-fasting conditions at a dose of 50 mg/body with water (30 mL) (n=4: 12.15 kg (5 years old), 13.00 kg (5 years old), 12.40 kg (5 years old), 12.95 kg (5 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

The same operation as the concentration measurement in (2) of Comparative Example PK1 was performed to calculate the plasma concentration of compound (I), and the plasma concentration transition data shown in Fig. 15 was obtained.

From Fig. 15, when the oral solid formulation produced by the formulation prescription of (Example 2-4) was orally administered, the maximum plasma concentration (577 nM) was reached 2 hours after administration, and the plasma concentration of compound (I) maintained a high concentration (plasma concentration 12 hours after administration: 189 nM, plasma concentration 24 hours after administration: 41.7 nM) even 12 hours after administration and beyond.

### (Example PK2-5)

### (1) Administration and blood collection of test formulation

An oral solid formulation (containing 50 mg of compound (I)) produced by the formulation prescription of Example 2-5 was orally administered to beagle dogs (male, 4 years old, 11.90 to 13.10 kg) under non-fasting conditions at a dose of 50 mg/body with water (30 mL) (n=4: 11.90 kg (4 years old), 13.10 kg (4 years old), 12.20 kg (4 years old), 12.80 kg (4 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

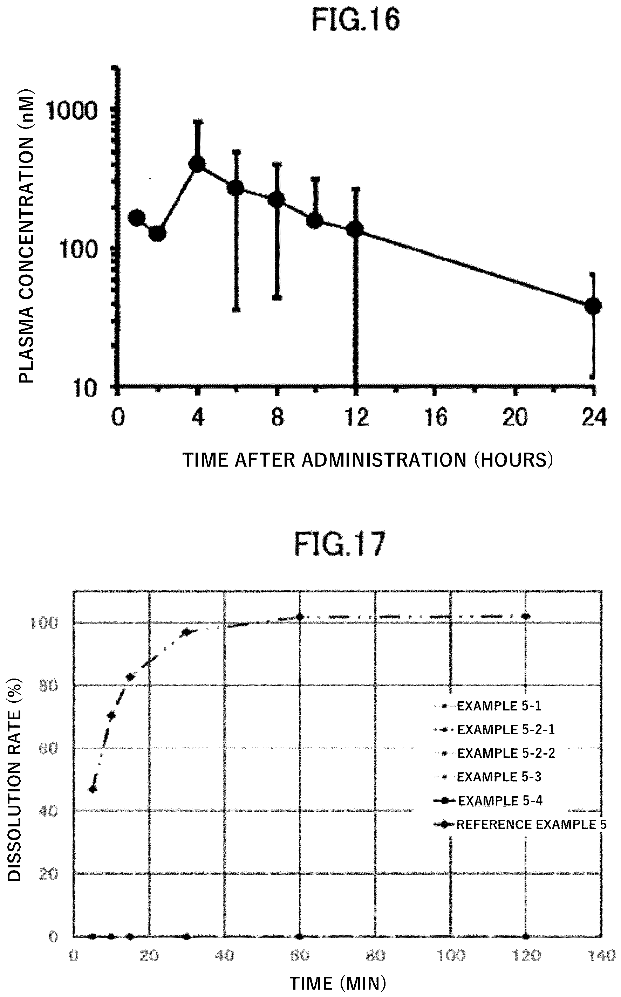

The same operation as the concentration measurement in (2) of Comparative Example PK1 was performed to calculate the plasma concentration of compound (I), and the plasma concentration transition data shown in Fig. 16 was obtained.

From Fig. 16, when the oral solid formulation produced by the formulation prescription of (Example 2-5) was orally administered, the maximum plasma concentration (397 nM) was reached 4 hours after administration, and the plasma concentration of compound (I) maintained a high concentration (plasma concentration 12 hours after administration: 135 nM, plasma concentration 24 hours after administration: 38.2 nM) even 12 hours after administration and beyond.

### <Production of Cores of Examples 5-1, 5-2-1, 5-2-2, 5-3, and 5-4, and Reference Example 5>

According to the formulation shown in Table 15 below, 44.0 g of light anhydrous silicic acid was added to 517.0 g of 1-((1R,2R)-2-hydroxy-4,4-dimethyl-1,2,3,4-tetrahydronaphthalen-1-yl)-3-(2-phenylpyridin-3-yl)urea and manually mixed using a polyethylene bag to obtain a mixture. 1408.0 g of mannitol, 11.0 g of talc, 88.0 g of croscarmellose sodium, and 110.0 g of crystalline cellulose were added to the obtained mixture, manually mixed using a polyethylene bag, sieved to break up agglomerates, and further 22.0 g of magnesium stearate was added and manually mixed to obtain a mixed powder for tableting. The obtained mixed powder for tableting was tableted using a table-top rotary tableting machine (PICCOLA NOVA, manufactured by RIVA S.A.) to obtain a core.

### <Production of Formulation (Tablet) of Example 5-1>

200 g of the core obtained in <Production of Cores of Examples 5-1, 5-2-1, 5-2-2, 5-3, and 5-4, and Reference Example 5> above was placed in a film coating machine (PRC-GTX-mini, manufactured by Powrex Corporation), and film-coated with a liquid in which carboxymethyl ethyl cellulose, triethyl citrate, talc, and titanium dioxide were dispersed or dissolved in anhydrous ethanol and purified water to obtain the formulation (tablet) of Example 5-1.

### <Production of Formulation (Tablet) of Example 5-2-1>

200 g of the core obtained in <Production of Cores of Examples 5-1, 5-2-1, 5-2-2, 5-3, and 5-4, and Reference Example 5> above was placed in a film coating machine (PRC-GTX-mini, manufactured by Powrex Corporation), and film-coated with a liquid in which methacrylic acid copolymer L, triethyl citrate, talc, and titanium dioxide were dispersed or dissolved in anhydrous ethanol and purified water to obtain the formulation (tablet) of Example 5-2-1.

### <Production of Formulation (Tablet) of Example 5-2-2>

200 g of the core obtained in <Production of Cores of Examples 5-1, 5-2-1, 5-2-2, 5-3, and 5-4, and Reference Example 5> above was placed in a film coating machine (PRC-GTX-mini, manufactured by Powrex Corporation), and film-coated with a liquid in which methacrylic acid copolymer L, triethyl citrate, talc, and titanium dioxide were dispersed or dissolved in anhydrous ethanol and purified water to obtain the formulation (tablet) of Example 5-2-2.

### <Production of Formulation (Tablet) of Example 5-3>

200 g of the core obtained in <Production of Cores of Examples 5-1, 5-2-1, 5-2-2, 5-3, and 5-4, and Reference Example 5> above was placed in a film coating machine (PRC-GTX-mini, manufactured by Powrex Corporation), and film-coated with a liquid in which cellulose acetate phthalate, triethyl citrate, talc, and titanium dioxide were dispersed or dissolved in anhydrous ethanol and purified water to obtain the formulation (tablet) of Example 5-3.

### <Production of Formulation (Tablet) of Example 5-4>

200 g of the core obtained in <Production of Cores of Examples 5-1, 5-2-1, 5-2-2, 5-3, and 5-4, and Reference Example 5> above was placed in a film coating machine (PRC-GTX-mini, manufactured by Powrex Corporation), and film-coated with a liquid in which methacrylic acid copolymer S, triethyl citrate, talc, and titanium dioxide were dispersed or dissolved in anhydrous ethanol and acetone to obtain the formulation (tablet) of Example 5-4.

### <Production of Formulation (Tablet) of Reference Example 5>

200 g of the core obtained in <Production of Cores of Examples 5-1, 5-2-1, 5-2-2, 5-3, and 5-4, and Reference Example 5> above was placed in a film coating machine (PRC-GTX-mini, manufactured by Powrex Corporation), and film-coated with a liquid in which aminoalkyl methacrylate copolymer E, triethyl citrate, talc, and titanium dioxide were dispersed or dissolved in anhydrous ethanol and purified water to obtain the formulation (tablet) of Reference Example 5.

**Table 15**

| Ingredient | Example 5-1 | Example 5-2-1 | Example 5-2-2 | Example 5-3 | Example 5-4 | Reference Example 5 |
|---|---|---|---|---|---|---|
| Compound (I) | 50.0 | 50.0 | 50.0 | 50.0 | 50.0 | 50.0 |
| Mannitol (Parteck M100) | 142.5 | 142.5 | 142.5 | 142.5 | 142.5 | 142.5 |
| Crystalline cellulose (Ceolus KG-1000) | 11 | 11 | 11 | 11 | 11 | 11 |
| Light anhydrous silicic acid | 4.4 | 4.4 | 4.4 | 4.4 | 4.4 | 4.4 |
| Talc | 1.1 | 1.1 | 1.1 | 1.1 | 1.1 | 1.1 |
| Croscarmellose sodium (Ac-Di-Sol) | 8.8 | 8.8 | 8.8 | 8.8 | 8.8 | 8.8 |
| Magnesium stearate | 2.2 | 2.2 | 2.2 | 2.2 | 2.2 | 2.2 |
| Core portion subtotal | 220 | 220 | 220 | 220 | 220 | 220 |
| Carboxymethyl ethyl cellulose (CMEC) | 7.9 | - | - | - | - | - |
| Methacrylic acid copolymer L (EUDRAGIT L100) | - | 7.9 | 5.5 | - | - | - |
| Cellulose acetate phthalate (Cellacefate) | - | - | - | 18.2 | - | - |
| Methacrylic acid copolymer S (EUDRAGIT S100) | - | - | - | - | 7.9 | - |
| Aminoalkyl methacrylate copolymer E | - | - | - | - | - | 7.9 |
| (EUDRAGIT E100) | | | | | | |
| Triethyl citrate | 1.6 | 1.6 | 1.1 | 3.7 | 1.6 | 1.6 |
| Talc | 1.6 | 1.6 | 1.1 | 3.7 | 1.6 | 1.6 |
| Titanium dioxide | 1.9 | 1.9 | 1.3 | 4.4 | 1.9 | 1.9 |
| Anhydrous ethanol | 0 | 0 | 0 | 0 | 0 | 0 |
| Purified water | 0 | 0 | 0 | 0 | 0 | 0 |
| Acetone | 0 | 0 | 0 | 0 | 0 | 0 |
| Coating portion subtotal | 13 | 13 | 9 | 30 | 13 | 13 |
| Total | 233 | 233 | 229 | 250 | 233 | 233 |

| | | | | | | |
|---|---|---|---|---|---|---|
| (The unit of numerical values is mg) | | | | | | |

### <Test Example 9> Dissolution Test (9)

For each formulation (tablet) produced in Examples 5-1, 5-2-1, 5-2-2, 5-3, and 5-4, and Reference Example 5, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 1 (JP1 solution) as the dissolution test solution, and a paddle rotation speed of 50 revolutions/minute. 5, 10, 15, 30, 60, and 120 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 16 and Fig. 17.

**Table 16**

| Formulation (tablets) | Sampling time (min) | | | | | |
|---|---|---|---|---|---|---|
| | 5 min | 10 min | 15 min | 30 min | 60 min | 120 min |
| Example 5-1 (%) | 0 | 0 | 0 | 0 | 0 | 0 |
| Example 5-2-1 (%) | 0 | 0 | 0 | 0 | 0 | 0 |
| Example 5-2-2 (%) | 0 | 0 | 0 | 0 | 0 | 0 |
| Example 5-3 (%) | 0 | 0 | 0 | 0 | 0 | 0 |
| Example 5-4 (%) | 0 | 0 | 0 | 0 | 0 | 0 |
| Reference Example 5 (%) | 46.9 | 70.4 | 82.7 | 97 | 101.7 | 102 |

### <Test Example 10> Dissolution Test (10)

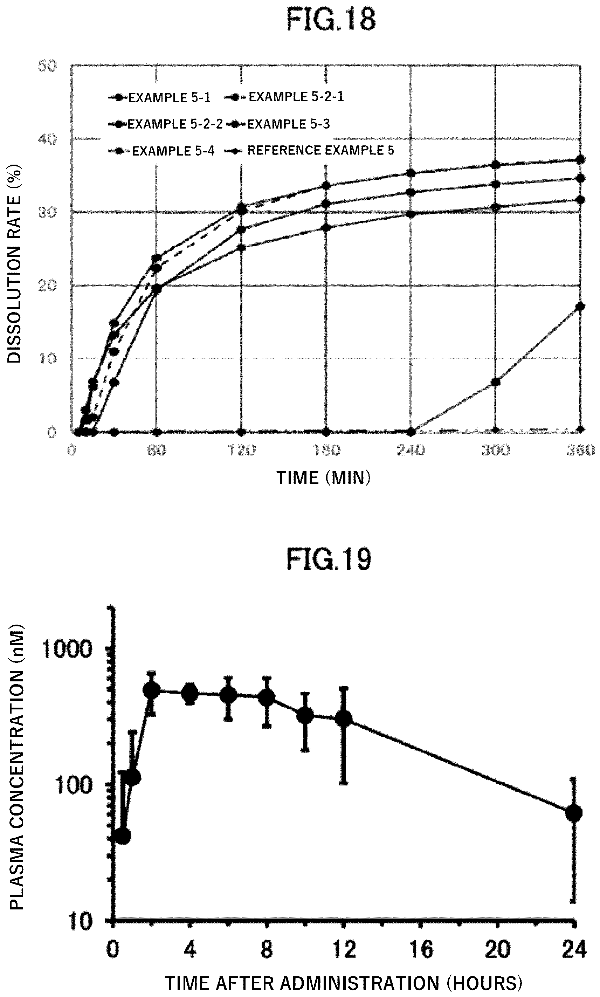

For each formulation (tablet) produced in Examples 5-1, 5-2-1, 5-2-2, 5-3, and 5-4, and Reference Example 5, a dissolution test was performed using a dissolution tester (NTR-8000AC series, NTR-8400AC series, NTR-8600AS series, manufactured by Toyama Sangyo Co., Ltd.) under the conditions of the paddle method of the dissolution test method described in Japanese Pharmacopoeia, Eighteenth Edition, Dissolution Test Solution 2 (JP2 solution) as the dissolution test solution, and a paddle rotation speed of 50 revolutions/minute. 5, 10, 15, 30, 60, 120, 180, 240, 300, and 360 minutes after the start of the test, the peak area of 1-((1R,2R)-2-hydroxy-4,4-dimethyl-1,2,3,4-tetrahydronaphthalen-1-yl)-3-(2-phenylpyridin-3-yl)urea in the dissolution test solution was measured using high performance liquid chromatography (HPLC method), and the dissolution rate was calculated by calculating the concentration of 1-((1R,2R)-2-hydroxy-4,4-dimethyl-1,2,3,4-tetrahydronaphthalen-1-yl)-3-(2-phenylpyridin-3-yl)urea from the obtained peak area. As to the HPLC method, measurement was performed using an HPLC system (manufactured by Shimadzu Corporation) (wavelength: 210 nm). The column used in the HPLC method was ZORBAX SB-Aq, inner diameter 4.6 mm, length 150 mm, particle size 3.5 µm (manufactured by Agilent), maintained at 40°C, and the mobile phase used was 0.02 mol/L phosphate buffer (pH 2.0)/acetonitrile mixture=1/1. The dissolution rates of 1-((1R,2R)-2-hydroxy-4,4-dimethyl-1,2,3,4-tetrahydronaphthalen-1-yl)-3-(2-phenylpyridin-3-yl)urea are shown in Table 17 and Fig. 18.

The dissolution test condition of JP2 solution (pH=6.8) is near the boundary of the dissolution pH in Example 5-4 using methacrylic acid copolymer S, which is a coating agent that dissolves around pH 7.0, and the dissolution of Example 5-4 was slightly slower than that of other Examples using a coating agent that dissolves at pH 6.5 or lower.

**Table 17**

| Formulation (tablets) | Sampling time (min) | | | | | | | | | |
|---|---|---|---|---|---|---|---|---|---|---|
| | 5 min | 10 min | 15 min | 30 min | 60 min | 120 min | 180 min | 240 min | 300 min | 360 min |
| Example 5-1 (%) | 0 | 3 | 6.8 | 13.2 | 19.6 | 25.1 | 27.8 | 29.7 | 30.7 | 31.7 |
| Example 5-2-1 (%) | 0 | 0.1 | 2 | 10.9 | 22.3 | 30.1 | 33.6 | 35.3 | 36.5 | 37.2 |
| Example 5-2-2 (%) | 0 | 1.5 | 6.1 | 14.8 | 23.7 | 30.7 | 33.6 | 35.3 | 36.4 | 37.1 |
| Example 5-3 (%) | 0 | 0 | 0 | 6.7 | 19.3 | 27.6 | 31.1 | 32.7 | 33.8 | 34.6 |
| Example 5-4 (%) | 0 | 0 | 0 | 0 | 0 | 0 | 0 | 0 | 6.7 | 17.1 |
| Reference Example 5 (%) | 0 | 0 | 0 | 0 | 0.1 | 0.1 | 0.2 | 0.2 | 0.3 | 0.4 |

Pharmacokinetics (PK) test:
Hereinafter, pharmacokinetics (PK) tests using the tablets (oral solid formulations) produced in Examples 5-1, 5-2-1, 5-3, and 5-4 will be described.

In the graphs of Figs. 19 to 22, the vertical axis represents the plasma concentration (blood concentration) (Plasma concentration) (nM) of compound (I), and the horizontal axis represents the time (Time after administration) (hours) after administration of compound (I).

### (Example PK5-1)

### (1) Administration and blood collection of test formulation

An oral solid formulation (containing 50.0 mg of compound (I)) produced by the formulation prescription of Example 5-1 was orally administered to beagle dogs (male, 5 years old, 11.5 to 13.1 kg) under non-fasting conditions at a dose of 50.0 mg/body with water (30 mL) (n=4: 11.5 kg (5 years old), 12.7 kg (5 years old), 12.1 kg (5 years old), 13.1 kg (5 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

To plasma (0.02 mL) collected in (1) above, an internal standard solution (0.18 mL) of Buspirone (manufactured by Sigma-Aldrich) dissolved in methanol at 50 nmol/L was added and thoroughly stirred, and then allowed to stand on ice for 30 minutes. The mixed solution was centrifuged at 540 G for 5 minutes (Hitachi multi-tube refrigerated centrifuge CF-9RX, manufactured by Hitachi Koki Co., Ltd.), the supernatant was collected, and the concentration of compound (I) in plasma was measured using LC-MS/MS (Triple Quad 4500, manufactured by AB Sciex). The plasma concentration of compound (I) was calculated from a calibration curve prepared using standard plasma samples prepared by serially diluting a 1 mg/mL solution of compound (I) with plasma, and the plasma concentration transition data shown in Fig. 19 was obtained.

From Fig. 19, when the oral solid formulation produced by the formulation prescription of (Example 5-1) was orally administered, the maximum plasma concentration (491 nM) was reached 2 hours after administration, and the plasma concentration of compound (I) maintained a high concentration (plasma concentration 12 hours after administration: 303 nM, plasma concentration 24 hours after administration: 61.5 nM) even 12 hours after administration and beyond.

### (Example PK5-2-1)

### (1) Administration and blood collection of test formulation

An oral solid formulation (containing 50.0 mg of compound (I)) produced by the formulation prescription of Example 5-2-1 was orally administered to beagle dogs (male, 5 years old, 11.35 to 13.25 kg) under non-fasting conditions at a dose of 50.0 mg/body with water (30 mL) (n=4: 11.35 kg (5 years old), 12.75 kg (5 years old), 12.20 kg (5 years old), 13.25 kg (5 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

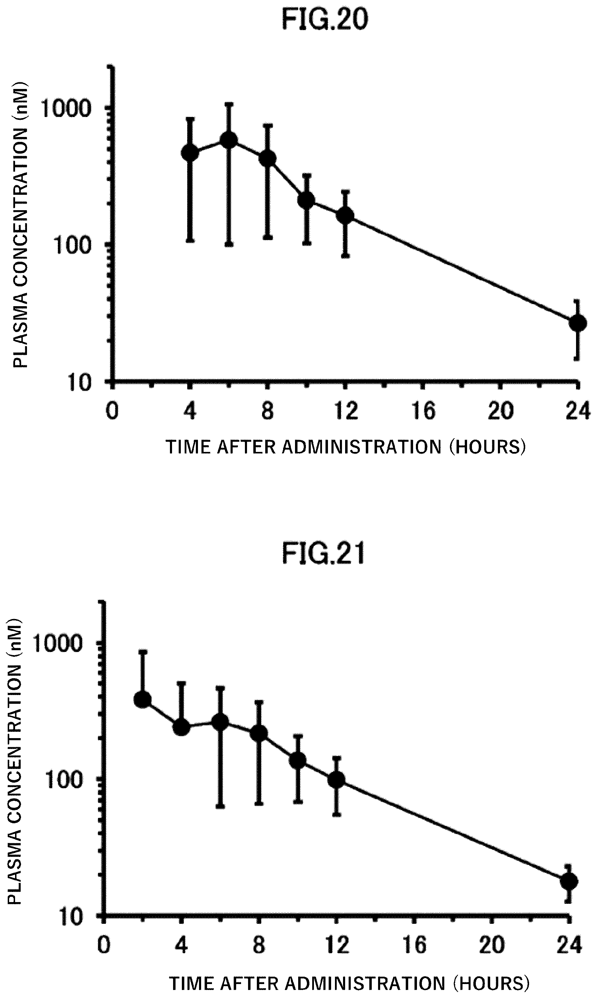

The same operation as the concentration measurement in (2) of Example PK5-1 was performed to calculate the plasma concentration of compound (I), and the plasma concentration transition data shown in Fig. 20 was obtained.

From Fig. 20, when the oral solid formulation produced by the formulation prescription of (Example 5-2-1) was orally administered, the maximum plasma concentration (581 nM) was reached 6 hours after administration, and the plasma concentration of compound (I) maintained a high concentration (plasma concentration 12 hours after administration: 163 nM, plasma concentration 24 hours after administration: 26.7 nM) even 12 hours after administration and beyond.

### (Example PK5-3)

### (1) Administration and blood collection of test formulation

An oral solid formulation (containing 50 mg of compound (I)) produced by the formulation prescription of Example 5-3 was orally administered to beagle dogs (male, 5 years old, 11.5 to 13.0 kg) under non-fasting conditions at a dose of 50 mg/body with water (30 mL) (n=4: 11.5 kg (5 years old), 12.8 kg (5 years old), 12.2 kg (5 years old), 13.0 kg (5 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

The same operation as the concentration measurement in (2) of Example PK5-1 was performed to calculate the plasma concentration of compound (I), and the plasma concentration transition data shown in Fig. 21 was obtained.

From Fig. 21, when the oral solid formulation produced by the formulation prescription of (Example 5-3) was orally administered, the maximum plasma concentration (381 nM) was reached 2 hours after administration, and the plasma concentration of compound (I) maintained a high concentration (plasma concentration 12 hours after administration: 98.6 nM, plasma concentration 24 hours after administration: 17.9 nM) even 12 hours after administration and beyond.

### (Example PK5-4)

### (1) Administration and blood collection of test formulation

An oral solid formulation (containing 50.0 mg of compound (I)) produced by the formulation prescription of Example 5-4 was orally administered to beagle dogs (male, 5 years old, 11.5 to 13.1 kg) under non-fasting conditions at a dose of 50.0 mg/body with water (30 mL) (n=4: 11.5 kg (5 years old), 12.7 kg (5 years old), 12.0 kg (5 years old), 13.1 kg (5 years old)). Blood (0.5 mL) was collected from the cephalic vein of the beagle dogs at 0.5, 1, 2, 4, 6, 8, 10, 12, and 24 hours after administration. Plasma was collected by centrifuging the collected blood (using High-Speed Refrigerated Micro Centrifuge MX-201, manufactured by Tomy Seiko Co., Ltd., 10,000×g, 5 minutes, 4°C).

### (2) Measurement of concentration of compound (I) in plasma

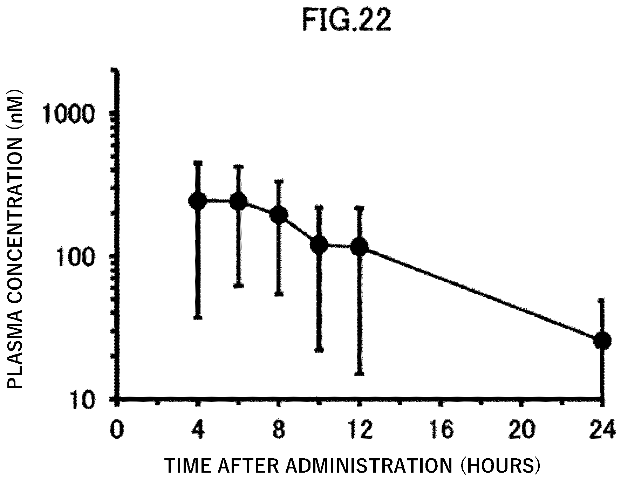

The same operation as the concentration measurement in (2) of Example PK5-1 was performed to calculate the plasma concentration of compound (I), and the plasma concentration transition data shown in Fig. 22 was obtained.

From Fig. 22, when the oral solid formulation produced by the formulation prescription of (Example 5-4) was orally administered, the maximum plasma concentration (243 nM) was reached 4 hours after administration, and the plasma concentration of compound (I) maintained a high concentration (plasma concentration 12 hours after administration: 116 nM, plasma concentration 24 hours after administration: 25.6 nM) even 12 hours after administration and beyond.

Other aspects of the present invention may be as follows.
[1] An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
   (1) a core containing the active ingredient; and
   (2) a coating layer covering the core, the coating layer containing at least one coating agent selected from the group consisting of hypromellose phthalate, methacrylic acid copolymer, hypromellose acetate succinate, cellulose acetate phthalate, and carboxymethyl ethyl cellulose.
[2] The oral solid formulation according to [1], wherein the methacrylic acid copolymer is at least one selected from the group consisting of methacrylic acid copolymer LD, methacrylic acid copolymer L, methacrylic acid copolymer S, and dry methacrylic acid copolymer LD.
[3] The oral solid formulation according to [1] or [2], wherein the coating layer further contains at least one plasticizer selected from the group consisting of triethyl citrate, tributyl citrate, polyethylene glycol, propylene glycol, sodium lauryl sulfate in ethanol, triacetin, and polysorbate 80.
[4] The oral solid formulation according to any one of [1] to [3], wherein the coating layer further contains at least one additive selected from the group consisting of titanium dioxide, talc, crystalline cellulose, calcium carbonate, and shellac.
[5] The oral solid formulation according to any one of [1] to [4], wherein the core further contains at least one additive selected from the group consisting of excipients, flow agents, disintegrants, and glidants.
[6] The oral solid formulation according to [5], wherein the excipient is at least one selected from the group consisting of glucose, lactose, granulated lactose, sucrose, white sugar, mannitol, mannite, xylitol, sorbitol, crystalline cellulose, microcrystalline cellulose, silicic acid, starch, maize starch, potato starch, dicalcium phosphate dihydrate, isomalt, anhydrous calcium phosphate, anhydrous calcium hydrogen phosphate, corn starch, pregelatinized starch, partially pregelatinized starch, light anhydrous silicic acid, and titanium dioxide.
[7] The oral solid formulation according to [5], wherein the flow agent is at least one selected from the group consisting of silicon dioxide, talc, hydrous silicon dioxide, magnesium aluminometasilicate, and granulated calcium hydrogen phosphate.
[8] The oral solid formulation according to [5], wherein the disintegrant is at least one selected from the group consisting of starches, sodium carboxymethyl starch, carmellose, carmellose calcium, croscarmellose sodium, crospovidone, sodium starch glycolate, sodium carboxymethyl cellulose, carboxymethyl cellulose calcium, polyvinylpyrrolidone, methylcellulose, microcrystalline cellulose, crystalline cellulose, low-alkyl-substituted hydroxypropyl cellulose, sodium alginate, corn starch, and sodium carboxymethyl starch.
[9] The oral solid formulation according to [5], wherein the glidant is at least one selected from the group consisting of magnesium stearate, calcium stearate, zinc stearate, sodium stearyl fumarate, a mixture of magnesium stearate and sodium lauryl sulfate, talc, carboxymethyl cellulose, stearic acid, sodium lauryl sulfate, carnauba wax, sucrose fatty acid ester, polyethylene glycol, glycerin, glyceryl monostearate, castor oil, and hydrogenated castor oil.
[10] The oral solid formulation according to any one of [1] to [9], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is 13% or more.
[11] The oral solid formulation according to any one of [1] to [9], wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 6 hours after the start of the dissolution test is 13% or more.
[12] The oral solid formulation according to any one of [1] to [11], wherein, when a dissolution test is performed using Dissolution Test Solution 1 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is 2.5% or less.
[13] The oral solid formulation according to any one of [1] to [12], wherein the coating layer dissolves in a solution having a pH in a range of 5.0 to 7.0.
[14] An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
   (1) a core containing the following components (a) to (e):
      (a) the active ingredient;
      (b) at least one excipient selected from the group consisting of crystalline cellulose, granulated lactose, and crystalline cellulose;
      (c) at least one flow agent selected from the group consisting of light anhydrous silicic acid, talc, and magnesium aluminometasilicate;
      (d) at least one disintegrant selected from croscarmellose sodium and sodium starch glycolate; and
      (e) at least one glidant selected from magnesium stearate and sodium stearyl fumarate; and
   (2) a coating layer covering the core, the coating layer containing at least one coating agent selected from the group consisting of hypromellose phthalate, methacrylic acid copolymer, hypromellose acetate succinate, cellulose acetate phthalate, and carboxymethyl ethyl cellulose.
[15] The oral solid formulation according to any one of [1] to [14], which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein the amount or equivalent amount means an amount at which, 12 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes 90 nM or more.
[16] The oral solid formulation according to any one of [1] to [14], which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein the amount or equivalent amount means an amount at which, either 12 hours or 24 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes 90 nM or more.
[17] The oral solid formulation according to [15] or [16], wherein the administration subject is a human or a non-human mammal.
[18] The oral solid formulation according to any one of [1] to [17], for use in prevention and/or treatment of a disease in which TrkA is involved.
[19] The oral solid formulation according to [18], wherein the disease in which TrkA is involved is pain or pruritus.

## Claims

1. An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
(1) a core containing the active ingredient; and
(2) a coating layer covering the core, the coating layer containing at least one coating agent selected from the group consisting of hypromellose phthalate, methacrylic acid copolymer, hypromellose acetate succinate, cellulose acetate phthalate, and carboxymethyl ethyl cellulose.

2. The oral solid formulation according to claim 1, wherein the methacrylic acid copolymer is at least one selected from the group consisting of methacrylic acid copolymer LD, methacrylic acid copolymer L, methacrylic acid copolymer S, and dry methacrylic acid copolymer LD.

3. The oral solid formulation according to claim 1 or 2, wherein the coating layer further contains at least one plasticizer selected from the group consisting of triethyl citrate, tributyl citrate, polyethylene glycol, propylene glycol, sodium lauryl sulfate in ethanol, triacetin, and polysorbate 80.

4. The oral solid formulation according to any one of claims 1 to 3, wherein the coating layer further contains at least one additive selected from the group consisting of titanium dioxide, talc, crystalline cellulose, calcium carbonate, and shellac.

5. The oral solid formulation according to any one of claims 1 to 4, wherein the core further contains at least one additive selected from the group consisting of excipients, flow agents, disintegrants, and glidants.

6. The oral solid formulation according to claim 5, wherein the excipient is at least one selected from the group consisting of glucose, lactose, granulated lactose, sucrose, white sugar, mannitol, mannite, xylitol, sorbitol, crystalline cellulose, microcrystalline cellulose, silicic acid, starch, maize starch, potato starch, dicalcium phosphate dihydrate, isomalt, anhydrous calcium phosphate, anhydrous calcium hydrogen phosphate, corn starch, pregelatinized starch, partially pregelatinized starch, light anhydrous silicic acid, and titanium dioxide.

7. The oral solid formulation according to claim 5, wherein the flow agent is at least one selected from the group consisting of silicon dioxide, talc, hydrous silicon dioxide, magnesium aluminometasilicate, and granulated calcium hydrogen phosphate.

8. The oral solid formulation according to claim 5, wherein the disintegrant is at least one selected from the group consisting of starches, sodium carboxymethyl starch, carmellose, carmellose calcium, croscarmellose sodium, crospovidone, sodium starch glycolate, sodium carboxymethyl cellulose, carboxymethyl cellulose calcium, polyvinylpyrrolidone, methylcellulose, microcrystalline cellulose, crystalline cellulose, low-alkyl-substituted hydroxypropyl cellulose, sodium alginate, corn starch, and sodium carboxymethyl starch.

9. The oral solid formulation according to claim 5, wherein the glidant is at least one selected from the group consisting of magnesium stearate, calcium stearate, zinc stearate, sodium stearyl fumarate, a mixture of magnesium stearate and sodium lauryl sulfate, talc, carboxymethyl cellulose, stearic acid, sodium lauryl sulfate, carnauba wax, sucrose fatty acid ester, polyethylene glycol, glycerin, glyceryl monostearate, castor oil, and hydrogenated castor oil.

10. The oral solid formulation according to any one of claims 1 to 9, wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is 13% or more.

11. The oral solid formulation according to any one of claims 1 to 9, wherein, when a dissolution test is performed using Dissolution Test Solution 2 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 6 hours after the start of the dissolution test is 13% or more.

12. The oral solid formulation according to any one of claims 1 to 9, wherein, when a dissolution test is performed using Dissolution Test Solution 1 in accordance with a paddle method of a dissolution test method described in Japanese Pharmacopoeia, Eighteenth Edition, a dissolution rate of 1-((1R,2R)-2-hydroxy-4,4-dimethyl-1,2,3,4-tetrahydronaphthalen-1-yl)-3-(2-phenylpyridin-3-yl)urea contained in the formulation 1 hour after the start of the dissolution test is 2.5% or less.

13. The oral solid formulation according to any one of claims 1 to 12, wherein the coating layer dissolves in a solution having a pH in a range of 5.0 to 7.0.

14. An oral solid formulation comprising 1-((1R,2R)-2-hydroxy-4,4-dimethyl-1,2,3,4-tetrahydronaphthalen-1-yl)-3-(2-phenylpyridin-3-yl)urea, or a pharmaceutically acceptable salt thereof, or a solvate thereof, as an active ingredient, wherein the formulation includes
(1) a core containing the following components (a) to (e):
(a) the active ingredient;
(b) at least one excipient selected from the group consisting of crystalline cellulose, granulated lactose, and crystalline cellulose;
(c) at least one flow agent selected from the group consisting of light anhydrous silicic acid, talc, and magnesium aluminometasilicate;
(d) at least one disintegrant selected from croscarmellose sodium and sodium starch glycolate; and
(e) at least one glidant selected from magnesium stearate and sodium stearyl fumarate; and
(2) a coating layer covering the core, the coating layer containing at least one coating agent selected from the group consisting of hypromellose phthalate, methacrylic acid copolymer, hypromellose acetate succinate, cellulose acetate phthalate, and carboxymethyl ethyl cellulose.

15. The oral solid formulation according to any one of claims 1 to 14, which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein the amount or equivalent amount means an amount at which, 12 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes 90 nM or more.

16. The oral solid formulation according to any one of claims 1 to 14, which contains 1-((1R,2R)-2-hydroxy-4,4-dimethyl-1,2,3,4-tetrahydronaphthalen-1-yl)-3-(2-phenylpyridin-3-yl)urea in an amount of 50 mg, or a pharmaceutically acceptable salt thereof in an amount equivalent to the amount, or a solvate thereof in an amount equivalent to the amount, wherein the amount or equivalent amount means an amount at which, either 12 hours or 24 hours after the formulation is initially administered to an administration subject, a concentration of 1-((1R,2R)-2-hydroxy-4,4-dimethyl-1,2,3,4-tetrahydronaphthalen-1-yl)-3-(2-phenylpyridin-3-yl)urea in a plasma of the subject becomes 90 nM or more.

17. The oral solid formulation according to claim 15 or 16, wherein the administration subject is a human or a non-human mammal.

18. The oral solid formulation according to any one of claims 1 to 17, for use in prevention and/or treatment of a disease in which TrkA is involved.

19. The oral solid formulation according to claim 18, wherein the disease in which TrkA is involved is pain or pruritus.
